# EUROPÄISCHE PATENTSCHRIFT

(11) **EP 2 704 594 B1**
(45) Veröffentlichungstag und Bekanntmachung des Hinweises auf die Patenterteilung: **27.04.2016**
(21) Anmeldenummer: 12702557.5
(22) Anmeldetag: 08.02.2012
(51) Int. Cl.: A23L 33/105, A23L 33/125, C12P 19/12, C12P 19/24, A23L 29/30

(54) **VERFAHREN ZUR HERSTELLUNG VON ISOMALTULOSE AUS PFLANZENSÄFTEN**
PROCESS FOR THE PRODUCTION OF ISOMALTULOSE FROM PLANT JUICES
PROCÉDÉ DE PRODUCTION D'ISOMALTULOSE À PARTIR DE JUS DE VÉGÉTAUX

(30) Priorität: 05.05.2011 DE 102011100772
(43) Veröffentlichungstag der Anmeldung: 12.03.2014
(73) Patentinhaber: Evonik Degussa GmbH, 45128 Essen (DE)
(72) Erfinder: HABERLAND, Jürgen, 45721 Haltern am See (DE); ZEHNACKER, Olivier, 44263 Dortmund (DE); GRAMMANN, Katrin, 45739 Oer-Erkenschwick (DE); HOLTKAMP, Manuel, 40764 Langenfeld-Richrath (DE); WOLTER, Jan, 40489 Düsseldorf (DE); HÜLLER, Thomas, 45772 Marl (DE); MOHR, Alexander, 45657 Recklinghausen (DE); GRIMBERG, Gerlind, 48301 Nottuln (DE); HAAS, Thomas, 48161 Münster (DE)
(86) Internationale Anmeldenummer: PCT/EP2012/052096
(87) Internationale Veröffentlichungsnummer: WO 2012/150051

(56) Entgegenhaltungen:
- EP-A1- 0 001 099
- EP-A1- 0 392 556
- US-A- 5 578 339

### SEQUENCE LISTING

<110> Evonik Degussa GmbH
<120> Verfahren zur Herstellung von Isomaltulose aus Pflanzensäften
<130> 201100086
<160> 16
<170> PatentIn version 3.4
<210> 1
   <211> 600
   <212> PRT
   <213> Erwinia rhapontici
<400> 1
   Met Ser Ser Gln Gly Leu Lys Thr Ala Val Ala Ile Phe Leu Ala Thr
   1               5                   10                  15
   Thr Phe Ser Ala Thr Ser Tyr Gln Ala Cys Ser Ala Gly Pro Asp Thr
               20                  25                  30
   Ala Pro Ser Leu Thr Val Gln Gln Ser Asn Ala Leu Pro Thr Trp Trp
           35                  40                  45
   Lys Gln Ala Val Phe Tyr Gln Val Tyr Pro Arg Ser Phe Lys Asp Thr
       50                  55                  60
   Asn Gly Asp Gly Ile Gly Asp Leu Asn Gly Ile Ile Glu Asn Leu Asp
   65                  70                  75                  80
   Tyr Leu Lys Lys Leu Gly Ile Asp Ala Ile Trp Ile Asn Pro His Tyr
                   85                  90                  95
   Asp Ser Pro Asn Thr Asp Asn Gly Tyr Asp Ile Arg Asp Tyr Arg Lys
               100                 105                 110
   Ile Met Lys Glu Tyr Gly Thr Met Glu Asp Phe Asp Arg Leu Ile Ser
           115                 120                 125
   Glu Met Lys Lys Arg Asn Met Arg Leu Met Ile Asp Ile Val Ile Asn
       130                 135                 140
   His Thr Ser Asp Gln His Ala Trp Phe Val Gln Ser Lys Ser Gly Lys
   145                 150                 155                 160
   Asn Asn Pro Tyr Arg Asp Tyr Tyr Phe Trp Arg Asp Gly Lys Asp Gly
                   165                 170                 175
   His Ala Pro Asn Asn Tyr Pro Ser Phe Phe Gly Gly Ser Ala Trp Glu
   180 185 190
   Lys Asp Asp Lys Ser Gly Gln Tyr Tyr Leu His Tyr Phe Ala Lys Gln
   195 200 205
   Gln Pro Asp Leu Asn Trp Asp Asn Pro Lys Val Arg Gln Asp Leu Tyr
   210 215 220
   Asp Met Leu Arg Phe Trp Leu Asp Lys Gly Val Ser Gly Leu Arg Phe
   225 230 235 240
   Asp Thr Val Ala Thr Tyr Ser Lys Ile Pro Asn Phe Pro Asp Leu Ser
   245 250 255
   Gln Gln Gln Leu Lys Asn Phe Ala Glu Glu Tyr Thr Lys Gly Pro Lys
   260 265 270
   Ile His Asp Tyr Val Asn Glu Met Asn Arg Glu Val Leu Ser His Tyr
   275 280 285
   Asp Ile Ala Thr Ala Gly Glu Ile Phe Gly Val Pro Leu Asp Lys Ser
   290 295 300
   Ile Lys Phe Phe Asp Arg Arg Arg Asn Glu Leu Asn Ile Ala Phe Thr
   305 310 315 320
   Phe Asp Leu Ile Arg Leu Asp Arg Asp Ala Asp Glu Arg Trp Arg Arg
   325 330 335
   Lys Asp Trp Thr Leu Ser Gln Phe Arg Lys Ile Val Asp Lys Val Asp
   340 345 350
   Gln Thr Ala Gly Glu Tyr Gly Trp Asn Ala Phe Phe Leu Asp Asn His
   355 360 365
   Asp Asn Pro Arg Ala Val Ser His Phe Gly Asp Asp Arg Pro Gln Trp
   370 375 380
   Arg Glu His Ala Ala Lys Ala Leu Ala Thr Leu Thr Leu Thr Gln Arg
   385 390 395 400
   Ala Thr Pro Phe Ile Tyr Gln Gly Ser Glu Leu Gly Met Thr Asn Tyr
   405 410 415
   Pro Phe Lys Lys Ile Asp Asp Phe Asp Asp Val Glu Val Lys Gly Phe
   420 425 430
   Trp Gln Asp Tyr Val Glu Thr Gly Lys Val Lys Ala Glu Glu Phe Leu
   435 440 445
   Gln Asn Val Arg Gln Thr Ser Arg Asp Asn Ser Arg Thr Pro Phe Gln
   450 455 460
   Trp Asp Ala Ser Lys Asn Ala Gly Phe Thr Ser Gly Thr Pro Trp Leu
   465 470 475 480
   Lys Ile Asn Pro Asn Tyr Lys Glu Ile Asn Ser Ala Asp Gln Ile Asn
   485 490 495
   Asn Pro Asn Ser Val Phe Asn Tyr Tyr Arg Lys Leu Ile Asn Ile Arg
   500 505 510
   His Asp Ile Pro Ala Leu Thr Tyr Gly Ser Tyr Ile Asp Leu Asp Pro
   515 520 525
   Asp Asn Asn Ser Val Tyr Ala Tyr Thr Arg Thr Leu Gly Ala Glu Lys
   530 535 540
   Tyr Leu Val Val Ile Asn Phe Lys Glu Glu Val Met His Tyr Thr Leu
   545 550 555 560
   Pro Gly Asp Leu Ser Ile Asn Lys Val Ile Thr Glu Asn Asn Ser His
   565 570 575
   Thr Ile Val Asn Lys Asn Asp Arg Gln Leu Arg Leu Glu Pro Trp Gln
   580 585 590
   Ser Gly Ile Tyr Lys Leu Asn Pro
   595 600
<210> 2
   <211> 584
   <212> PRT
   <213> Pseudomonas mesoacidophila
<400> 2
   Met Leu Met Lys Arg Leu Phe Ala Ala Ser Leu Met Leu Ala Phe Ser
   1 5 10 15
   Ser Val Ser Ser Val Arg Ala Glu Glu Ala Val Lys Pro Gly Ala Pro
   20 25 30
   Trp Trp Lys Ser Ala Val Phe Tyr Gln Val Tyr Pro Arg Ser Phe Lys
   35 40 45
   Asp Thr Asn Gly Asp Gly Ile Gly Asp Phe Lys Gly Leu Thr Glu Lys
       50                  55                  60
   Leu Asp Tyr Leu Lys Gly Leu Gly Ile Asp Ala Ile Trp Ile Asn Pro
   65                  70                  75                  80
   His Tyr Ala Ser Pro Asn Thr Asp Asn Gly Tyr Asp Ile Ser Asp Tyr
                   85                  90                  95
   Arg Glu Val Met Lys Glu Tyr Gly Thr Met Glu Asp Phe Asp Arg Leu
               100                 105                 110
   Met Ala Glu Leu Lys Lys Arg Gly Met Arg Leu Met Val Asp Val Val
           115                 120                 125
   Ile Asn His Ser Ser Asp Gln His Glu Trp Phe Lys Ser Ser Arg Ala
       130                 135                 140
   Ser Lys Asp Asn Pro Tyr Arg Asp Tyr Tyr Phe Trp Arg Asp Gly Lys
   145                 150                 155                 160
   Asp Gly His Glu Pro Asn Asn Tyr Pro Ser Phe Phe Gly Gly Ser Ala
                   165                 170                 175
   Trp Glu Lys Asp Pro Val Thr Gly Gln Tyr Tyr Leu His Tyr Phe Gly
               180                 185                 190
   Arg Gln Gln Pro Asp Leu Asn Trp Asp Thr Pro Lys Leu Arg Glu Glu
           195                 200                 205
   Leu Tyr Ala Met Leu Arg Phe Trp Leu Asp Lys Gly Val Ser Gly Met
       210                 215                 220
   Arg Phe Asp Thr Val Ala Thr Tyr Ser Lys Thr Pro Gly Phe Pro Asp
   225                 230                 235                 240
   Leu Thr Pro Glu Gln Met Lys Asn Phe Ala Glu Ala Tyr Thr Gln Gly
                   245                 250                 255
   Pro Asn Leu His Arg Tyr Leu Gln Glu Met His Glu Lys Val Phe Asp
               260                 265                 270
   His Tyr Asp Ala Val Thr Ala Gly Glu Ile Phe Gly Ala Pro Leu Asn
           275                 280                 285
   Gln Val Pro Leu Phe Ile Asp Ser Arg Arg Lys Glu Leu Asp Met Ala
       290                 295                 300
   Phe Thr Phe Asp Leu Ile Arg Tyr Asp Arg Ala Leu Asp Arg Trp His
   305                 310                 315                 320
   Thr Ile Pro Arg Thr Leu Ala Asp Phe Arg Gln Thr Ile Asp Lys Val
                   325                 330                 335
   Asp Ala Ile Ala Gly Glu Tyr Gly Trp Asn Thr Phe Phe Leu Gly Asn
               340                 345                 350
   His Asp Asn Pro Arg Ala Val Ser His Phe Gly Asp Asp Arg Pro Gln
           355                 360                 365
   Trp Arg Glu Ala Ser Ala Lys Ala Leu Ala Thr Val Thr Leu Thr Gln
       370                 375                 380
   Arg Gly Thr Pro Phe Ile Phe Gln Gly Asp Glu Leu Gly Met Thr Asn
   385                 390                 395                 400
   Tyr Pro Phe Lys Thr Leu Gln Asp Phe Asp Asp Ile Glu Val Lys Gly
                   405                 410                 415
   Phe Phe Gln Asp Tyr Val Glu Thr Gly Lys Ala Thr Ala Glu Glu Leu
               420                 425                 430
   Leu Thr Asn Val Ala Leu Thr Ser Arg Asp Asn Ala Arg Thr Pro Phe
           435                 440                 445
   Gln Trp Asp Asp Ser Ala Asn Ala Gly Phe Thr Thr Gly Lys Pro Trp
       450                 455                 460
   Leu Lys Val Asn Pro Asn Tyr Thr Glu Ile Asn Ala Ala Arg Glu Ile
   465                 470                 475                 480
   Gly Asp Pro Lys Ser Val Tyr Ser Phe Tyr Arg Asn Leu Ile Ser Ile
                   485                 490                 495
   Arg His Glu Thr Pro Ala Leu Ser Thr Gly Ser Tyr Arg Asp Ile Asp
               500                 505                 510
   Pro Ser Asn Ala Asp Val Tyr Ala Tyr Thr Arg Ser Gln Asp Gly Glu
           515                 520                 525
   Thr Tyr Leu Val Val Val Asn Phe Lys Ala Glu Pro Arg Ser Phe Thr
       530                 535                 540
   Leu Pro Asp Gly Met His Ile Ala Glu Thr Leu Ile Glu Ser Ser Ser
   545 550 555 560
   Pro Ala Ala Pro Ala Ala Gly Ala Ala Ser Leu Glu Leu Gln Pro Trp
   565 570 575
   Gln Ser Gly Ile Tyr Lys Val Lys
   580
<210> 3
   <211> 598
   <212> PRT
   <213> Pantoea dispersa
<400> 3
   Met Phe Leu Asn Gly Phe Lys Thr Val Ile Ala Leu Thr Met Ala Ser
   1               5                   10                  15
   Ser Phe Tyr Leu Ala Ala Ser Pro Leu Thr Lys Pro Ser Thr Pro Ile
               20                  25                  30
   Ala Ala Thr Asn Ile Gln Lys Ser Ala Asp Phe Pro Ile Trp Trp Lys
           35                  40                  45
   Gln Ala Val Phe Tyr Gln Ile Tyr Pro Arg Ser Phe Lys Asp Ser Asn
       50                  55                  60
   Gly Asp Gly Ile Gly Asp Ile Pro Gly Ile Ile Glu Lys Leu Asp Tyr
   65                  70                  75                  80
   Leu Lys Met Leu Gly Val Asp Ala Ile Trp Ile Asn Pro His Tyr Glu
                   85                  90                  95
   Ser Pro Asn Thr Asp Asn Gly Tyr Asp Ile Ser Asp Tyr Arg Lys Ile
               100                 105                 110
   Met Lys Glu Tyr Gly Ser Met Ala Asp Phe Asp Arg Leu Val Ala Glu
           115                 120                 125
   Met Asn Lys Arg Gly Met Arg Leu Met Ile Asp Ile Val Ile Asn His
       130                 135                 140
   Thr Ser Asp Arg His Arg Trp Phe Val Gln Ser Arg Ser Gly Lys Asp
   145                 150                 155                 160
   Asn Pro Tyr Arg Asp Tyr Tyr Phe Trp Arg Asp Gly Lys Gln Gly Gln
                   165                 170                 175
   Ala Pro Asn Asn Tyr Pro Ser Phe Phe Gly Gly Ser Ala Trp Gln Leu
               180                 185                 190
   Asp Lys Gln Thr Asp Gln Tyr Tyr Leu His Tyr Phe Ala Pro Gln Gln
           195                 200                 205
   Pro Asp Leu Asn Trp Asp Asn Pro Lys Val Arg Ala Glu Leu Tyr Asp
       210                 215                 220
   Ile Leu Arg Phe Trp Leu Asp Lys Gly Val Ser Gly Leu Arg Phe Asp
   225                 230                 235                 240
   Thr Val Ala Thr Phe Ser Lys Ile Pro Gly Phe Pro Asp Leu Ser Lys
                   245                 250                 255
   Ala Gln Leu Lys Asn Phe Ala Glu Ala Tyr Thr Glu Gly Pro Asn Ile
               260                 265                 270
   His Lys Tyr Ile His Glu Met Asn Arg Gln Val Leu Ser Lys Tyr Asn
           275                 280                 285
   Val Ala Thr Ala Gly Glu Ile Phe Gly Val Pro Val Ser Ala Met Pro
       290                 295                 300
   Asp Tyr Phe Asp Arg Arg Arg Glu Glu Leu Asn Ile Ala Phe Thr Phe
   305                 310                 315                 320
   Asp Leu Ile Arg Leu Asp Arg Tyr Pro Asp Gln Arg Trp Arg Arg Lys
                   325                 330                 335
   Pro Trp Thr Leu Ser Gln Phe Arg Gln Val Ile Ser Gln Thr Asp Arg
               340                 345                 350
   Ala Ala Gly Glu Phe Gly Trp Asn Ala Phe Phe Leu Asp Asn His Asp
           355                 360                 365
   Asn Pro Arg Gln Val Ser His Phe Gly Asp Asp Ser Pro Gln Trp Arg
       370                 375                 380
   Glu Arg Ser Ala Lys Ala Leu Ala Thr Leu Leu Leu Thr Gln Arg Ala
   385                 390                 395                 400
   Thr Pro Phe Ile Phe Gln Gly Ala Glu Leu Gly Met Thr Asn Tyr Pro
                   405                 410                 415
   Phe Lys Asn Ile Glu Glu Phe Asp Asp Ile Glu Val Lys Gly Phe Trp
               420                 425                 430
   Asn Asp Tyr Val Ala Ser Gly Lys Val Asn Ala Ala Glu Phe Leu Gln
   435 440 445
   Glu Val Arg Met Thr Ser Arg Asp Asn Ser Arg Thr Pro Met Gln Trp
   450 455 460
   Asn Asp Ser Val Asn Ala Gly Phe Thr Gln Gly Lys Pro Trp Phe His
   465 470 475 480
   Leu Asn Pro Asn Tyr Lys Gln Ile Asn Ala Ala Arg Glu Val Asn Lys
   485 490 495
   Pro Asp Ser Val Phe Ser Tyr Tyr Arg Gln Leu Ile Asn Leu Arg His
   500 505 510
   Gln Ile Pro Ala Leu Thr Ser Gly Glu Tyr Arg Asp Leu Asp Pro Gln
   515 520 525
   Asn Asn Gln Val Tyr Ala Tyr Thr Arg Ile Leu Asp Asn Glu Lys Tyr
   530 535 540
   Leu Val Val Val Asn Phe Lys Pro Glu Gln Leu His Tyr Ala Leu Pro
   545 550 555 560
   Asp Asn Leu Thr Ile Ala Ser Ser Leu Leu Glu Asn Val His Gln Pro
   565 570 575
   Ser Leu Gln Glu Asn Ala Ser Thr Leu Thr Leu Ala Pro Trp Gln Ala
   580 585 590
   Gly Ile Tyr Lys Leu Asn
   595
<210> 4
   <211> 598
   <212> PRT
   <213> Klebsiella pneumoniae
<400> 4
   Met Ser Phe Ile Thr Leu Arg Thr Gly Val Ala Val Ala Leu Ser Ser
   1 5 10 15
   Leu Met Ile Ser Leu Ala Ser Pro Ala Val Ser Ala Ala Pro Ser Val
   20 25 30
   Asn Gln Asp Ile Leu Val Gln Lys Glu Ser Glu Tyr Pro Ala Trp Trp
   35 40 45
   Lys Glu Ala Val Phe Tyr Gln Ile Tyr Pro Arg Ser Phe Lys Asp Thr
       50                  55                  60
   Asn Asp Asp Gly Ile Gly Asp Ile Arg Gly Ile Ile Glu Lys Leu Asp
   65                  70                  75                  80
   Tyr Leu Lys Ser Leu Gly Ile Asp Ala Ile Trp Ile Asn Pro His Tyr
                   85                  90                  95
   Asp Ser Pro Asn Thr Asp Asn Gly Tyr Asp Ile Ser Asn Tyr Arg Gln
               100                 105                 110
   Ile Met Lys Glu Tyr Gly Thr Met Glu Asp Phe Asp Asn Leu Val Ala
           115                 120                 125
   Glu Met Lys Lys Arg Asn Met Arg Leu Met Ile Asp Val Val Ile Asn
       130                 135                 140
   His Thr Ser Asp Gln His Pro Trp Phe Ile Gln Ser Lys Ser Asp Lys
   145                 150                 155                 160
   Asn Asn Pro Tyr Arg Asp Tyr Tyr Phe Trp Arg Asp Gly Lys Asp Asn
                   165                 170                 175
   Gln Pro Pro Asn Asn Tyr Pro Ser Phe Phe Gly Gly Ser Ala Trp Gln
               180                 185                 190
   Lys Asp Ala Lys Ser Gly Gln Tyr Tyr Leu His Tyr Phe Ala Arg Gln
           195                 200                 205
   Gln Pro Asp Leu Asn Trp Asp Asn Pro Lys Val Arg Glu Asp Leu Tyr
       210                 215                 220
   Ala Met Leu Arg Phe Trp Leu Asp Lys Gly Val Ser Gly Met Arg Phe
   225                 230                 235                 240
   Asp Thr Val Ala Thr Tyr Ser Lys Ile Pro Gly Phe Pro Asn Leu Thr
                   245                 250                 255
   Pro Glu Gln Gln Lys Asn Phe Ala Glu Gln Tyr Thr Met Gly Pro Asn
               260                 265                 270
   Ile His Arg Tyr Ile Gln Glu Met Asn Arg Glu Val Leu Ser Arg Tyr
           275                 280                 285
   Asp Val Ala Thr Ala Gly Glu Ile Phe Gly Val Pro Leu Asp Arg Ser
       290                 295                 300
   Ser Gln Phe Phe Asp Arg Arg Arg His Glu Leu Asn Met Ala Phe Met
   305                 310                 315                 320
   Phe Asp Leu Ile Arg Leu Asp Arg Asp Ser Asn Glu Arg Trp Arg His
                   325                 330                 335
   Lys Ser Trp Ser Leu Ser Gln Phe Arg Gln Ile Ile Ser Lys Met Asp
               340                 345                 350
   Val Thr Val Gly Lys Tyr Gly Trp Asn Thr Phe Phe Leu Asp Asn His
           355                 360                 365
   Asp Asn Pro Arg Ala Val Ser His Phe Gly Asp Asp Arg Pro Gln Trp
       370                 375                 380
   Arg Glu Ala Ser Ala Lys Ala Leu Ala Thr Ile Thr Leu Thr Gln Arg
   385                 390                 395                 400
   Ala Thr Pro Phe Ile Tyr Gln Gly Ser Glu Leu Gly Met Ile Asn Tyr
                   405                 410                 415
   Pro Phe Arg Gln Leu Asn Glu Phe Asp Asp Ile Glu Val Lys Gly Phe
               420                 425                 430
   Trp Gln Asp Tyr Val Gln Ser Gly Lys Val Thr Ala Thr Glu Phe Leu
           435                 440                 445
   Asp Asn Val Arg Leu Thr Ser Arg Asp Asn Ser Arg Thr Pro Phe Gln
       450                 455                 460
   Trp Asn Asp Thr Leu Asn Ala Gly Phe Thr Arg Gly Lys Pro Trp Phe
   465                 470                 475                 480
   His Ile Asn Pro Asn Tyr Val Glu Ile Asn Ala Glu Arg Glu Glu Thr
                   485                 490                 495
   Arg Glu Asp Ser Val Leu Asn Tyr Tyr Lys Lys Met Ile Gln Leu Arg
               500                 505                 510
   His His Ile Pro Ala Leu Val Tyr Gly Ala Tyr Gln Asp Leu Asn Pro
           515                 520                 525
   Gln Asp Asn Thr Val Tyr Ala Tyr Thr Arg Thr Leu Gly Asn Glu Arg
       530                 535                 540
   Tyr Leu Val Val Val Asn Phe Lys Glu Tyr Pro Val Arg Tyr Thr Leu
   545                 550                 555                 560
   Pro Ala Asn Asp Ala Ile Glu Glu Val Val Ile Asp Thr Gln Gln Gln
                   565                 570                 575
   Ala Thr Ala Pro His Ser Thr Ser Leu Ser Leu Ser Pro Trp Gln Ala
               580                 585                 590
   Gly Val Tyr Lys Leu Arg
           595
<210> 5
   <211> 558
   <212> PRT
   <213> Protaminobacter Rubrum
<400> 5
   Thr Ile Pro Lys Trp Trp Lys Glu Ala Val Phe Tyr Gln Val Tyr Pro
   1               5                   10                  15
   Arg Ser Phe Lys Asp Thr Asn Gly Asp Gly Ile Gly Asp Ile Asn Gly
               20                  25                  30
   Ile Ile Glu Lys Leu Asp Tyr Leu Lys Ala Leu Gly Ile Asp Ala Ile
           35                  40                  45
   Trp Ile Asn Pro His Tyr Asp Ser Pro Asn Thr Asp Asn Gly Tyr Asp
       50                  55                  60
   Ile Arg Asp Tyr Arg Lys Ile Met Lys Glu Tyr Gly Thr Met Glu Asp
   65                  70                  75                  80
   Phe Asp Arg Leu Ile Ser Glu Met Lys Lys Arg Asn Met Arg Leu Met
                   85                  90                  95
   Ile Asp Val Val Ile Asn His Thr Ser Asp Gln Asn Glu Trp Phe Val
               100                 105                 110
   Lys Ser Lys Ser Ser Lys Asp Asn Pro Tyr Arg Gly Tyr Tyr Phe Trp
           115                 120                 125
   Lys Asp Ala Lys Glu Gly Gln Ala Pro Asn Asn Tyr Pro Ser Phe Phe
       130                 135                 140
   Gly Gly Ser Ala Trp Gln Lys Asp Glu Lys Thr Asn Gln Tyr Tyr Leu
   145                 150                 155                 160
   His Tyr Phe Ala Lys Gln Gln Pro Asp Leu Asn Trp Asp Asn Pro Lys
                   165                 170                 175
   Val Arg Gln Asp Leu Tyr Ala Met Leu Arg Phe Trp Leu Asp Lys Gly
               180                 185                 190
   Val Ser Gly Leu Arg Phe Asp Thr Val Ala Thr Tyr Ser Lys Ile Pro
           195                 200                 205
   Asp Phe Pro Asn Leu Thr Gln Gln Gln Leu Lys Asn Phe Ala Ala Glu
       210                 215                 220
   Tyr Thr Lys Gly Pro Asn Ile His Arg Tyr Val Asn Glu Met Asn Lys
   225                 230                 235                 240
   Glu Val Leu Ser His Tyr Asp Ile Ala Thr Ala Gly Glu Ile Phe Gly
                   245                 250                 255
   Val Pro Leu Asp Gln Ser Ile Lys Phe Phe Asp Arg Arg Arg Asp Glu
               260                 265                 270
   Leu Asn Ile Ala Phe Thr Phe Asp Leu Ile Arg Leu Asp Arg Asp Ser
           275                 280                 285
   Asp Gln Arg Trp Arg Arg Lys Asp Trp Lys Leu Ser Gln Phe Arg Gln
       290                 295                 300
   Ile Ile Asp Asn Val Asp Arg Thr Ala Gly Glu Tyr Gly Trp Asn Ala
   305                 310                 315                 320
   Phe Phe Leu Asp Asn His Asp Asn Pro Arg Ala Val Ser His Phe Gly
                   325                 330                 335
   Asp Asp Arg Pro Gln Trp Arg Glu Pro Ser Ala Lys Ala Leu Ala Thr
               340                 345                 350
   Leu Thr Leu Thr Gln Arg Ala Thr Pro Phe Ile Tyr Gln Gly Ser Glu
           355                 360                 365
   Leu Gly Met Thr Asn Tyr Pro Phe Lys Ala Ile Asp Glu Phe Asp Asp
       370                 375                 380
   Ile Glu Val Lys Gly Phe Trp His Asp Tyr Val Glu Thr Gly Lys Val
   385                 390                 395                 400
   Lys Ala Asp Glu Phe Leu Gln Asn Val Arg Leu Thr Ser Arg Asp Asn
                   405                 410                 415
   Ser Arg Thr Pro Phe Gln Trp Asp Gly Ser Lys Asn Ala Gly Phe Thr
                   420                 425                 430
   Ser Gly Lys Pro Trp Phe Lys Val Asn Pro Asn Tyr Gln Glu Ile Asn
           435                 440                 445
   Ala Val Ser Gln Val Thr Gln Pro Asp Ser Val Phe Asn Tyr Tyr Arg
       450                 455                 460
   Gln Leu Ile Lys Ile Arg His Asp Ile Pro Ala Leu Thr Tyr Gly Thr
   465                 470                 475                 480
   Tyr Thr Asp Leu Asp Pro Ala Asn Asp Ser Val Tyr Ala Tyr Thr Arg
                   485                 490                 495
   Ser Leu Gly Ala Glu Lys Tyr Leu Val Val Val Asn Phe Lys Glu Gln
               500                 505                 510
   Met Met Arg Tyr Lys Leu Pro Asp Asn Leu Ser Ile Glu Lys Val Ile
           515                 520                 525
   Ile Asp Ser Asn Ser Lys Asn Val Val Lys Lys Asn Asp Ser Leu Leu
       530                 535                 540
   Glu Leu Lys Pro Trp Gln Ser Gly Val Tyr Lys Leu Asn Gln
   545                 550                 555
<210> 6
   <211> 629
   <212> PRT
   <213> Protaminobacter Rubrum
<400> 6
   Met Pro Arg Gln Gly Leu Lys Thr Ala Leu Ala Ile Phe Leu Thr Thr
   1               5                   10                  15
   Ser Leu Cys Ile Ser Cys Gln Gln Ala Phe Gly Thr Gln Gln Pro Leu
               20                  25                  30
   Leu Asn Glu Lys Ser Ile Glu Gln Ser Lys Thr Ile Pro Lys Trp Trp
           35                  40                  45
   Lys Glu Ala Val Phe Tyr Gln Val Tyr Pro Arg Ser Phe Lys Asp Thr
       50                  55                  60
   Asn Gly Asp Gly Ile Gly Asp Ile Asn Gly Ile Ile Glu Lys Leu Asp
   65                  70                  75                  80
   Tyr Leu Lys Ala Leu Gly Ile Asp Ala Ile Trp Ile Asn Pro His Tyr
   85 90 95
   Asp Ser Pro Asn Thr Asp Asn Gly Tyr Asp Ile Arg Asp Tyr Arg Lys
   100 105 110
   Ile Met Lys Glu Tyr Gly Thr Met Glu Asp Phe Asp Arg Leu Ile Ser
   115 120 125
   Glu Met Lys Lys Arg Asn Met Arg Leu Met Ile Asp Val Val Ile Asn
   130 135 140
   His Thr Ser Asp Gln Asn Glu Trp Phe Val Lys Ser Lys Ser Ser Lys
   145 150 155 160
   Asp Asn Pro Tyr Arg Gly Tyr Tyr Phe Trp Lys Asp Ala Lys Glu Gly
   165 170 175
   Gln Ala Pro Asn Asn Tyr Pro Ser Phe Phe Gly Gly Ser Ala Trp Gln
   180 185 190
   Lys Asp Glu Lys Thr Asn Gln Tyr Tyr Leu His Tyr Phe Ala Lys Gln
   195 200 205
   Gln Pro Asp Leu Asn Trp Asp Asn Pro Lys Val Arg Gln Asp Leu Tyr
   210 215 220
   Ala Met Leu Arg Phe Trp Leu Asp Lys Gly Val Ser Gly Leu Arg Phe
   225 230 235 240
   Asp Thr Val Ala Thr Tyr Ser Lys Ile Pro Asp Phe Pro Asn Leu Thr
   245 250 255
   Gln Gln Gln Leu Lys Asn Phe Ala Ala Glu Tyr Thr Lys Gly Pro Asn
   260 265 270
   Ile His Arg Tyr Val Asn Glu Met Asn Lys Glu Val Leu Ser His Tyr
   275 280 285
   Asp Ile Ala Thr Ala Gly Glu Ile Phe Gly Val Pro Leu Asp Gln Ser
   290 295 300
   Ile Lys Phe Phe Asp Arg Arg Arg Asp Glu Leu Asn Ile Ala Phe Thr
   305 310 315 320
   Phe Asp Leu Ile Arg Leu Asp Arg Asp Ser Asp Gln Arg Trp Arg Arg
   325 330 335
   Lys Asp Trp Lys Leu Ser Gln Phe Arg Gln Ile Ile Asp Asn Val Asp
               340                 345                 350
   Arg Thr Ala Gly Glu Tyr Gly Trp Asn Ala Phe Phe Leu Asp Asn His
           355                 360                 365
   Asp Asn Pro Arg Ala Val Ser His Phe Gly Asp Asp Asp Arg Pro Gln
       370                 375                 380
   Trp Arg Glu Pro Ser Ala Lys Ala Leu Ala Thr Leu Thr Leu Thr Gln
   385                 390                 395                 400
   Arg Ala Thr Pro Phe Ile Tyr Gln Gly Ser Glu Leu Gly Met Thr Asn
                   405                 410                 415
   Tyr Pro Phe Lys Ala Ile Asp Glu Phe Asp Asp Ile Glu Val Lys Gly
               420                 425                 430
   Phe Trp His Asp Tyr Val Glu Thr Gly Lys Val Lys Ala Asp Glu Phe
           435                 440                 445
   Leu Gln Asn Val Arg Leu Thr Ser Arg Asp Asn Ser Arg Thr Pro Phe
       450                 455                 460
   Gln Trp Asp Gly Ser Lys Asn Ala Gly Phe Thr Ser Gly Lys Pro Trp
   465                 470                 475                 480
   Phe Lys Val Asn Pro Asn Tyr Gln Glu Ile Asn Ala Val Ser Gln Val
                   485                 490                 495
   Thr Gln Pro Asp Ser Val Phe Asn Tyr Tyr Arg Gln Leu Ile Lys Ile
               500                 505                 510
   Arg His Asp Ile Pro Ala Leu Thr Tyr Gly Thr Tyr Thr Asp Leu Asp
           515                 520                 525
   Pro Ala Asn Asp Ser Val Tyr Ala Tyr Thr Arg Ser Leu Gly Ala Glu
       530                 535                 540
   Lys Tyr Leu Val Val Val Asn Phe Lys Glu Gln Met Met Arg Tyr Lys
   545                 550                 555                 560
   Leu Pro Asp Asn Leu Ser Ile Glu Lys Val Ile Ile Asp Ser Asn Ser
                   565                 570                 575
   Lys Asn Val Val Lys Lys Asn Asp Ser Leu Leu Glu Leu Lys Pro Trp
               580                 585                 590
   Gln Ser Gly Val Tyr Lys Thr Lys Ser Ile Asn Leu Ile Val Thr Pro
   595 600 605
   Asn Asn Val Asn Ile Leu Lys Leu Leu Lys Pro Ala Phe Tyr Ala Gly
   610 615 620
   Phe Phe Ser Ala Lys
   625
<210> 7
   <211> 600
   <212> PRT
   <213> Serratia odorifera
<400> 7
   Met Pro Arg Gln Gly Leu Lys Thr Ala Leu Ala Ile Phe Leu Thr Thr
   1 5 10 15
   Ser Leu Ser Val Ser Cys Gln Gln Ala Leu Gly Thr Gln Gln Pro Leu
   20 25 30
   Leu Asn Glu Lys Ser Ile Glu Gln Ser Lys Thr Ile Pro Lys Trp Trp
   35 40 45
   Lys Glu Ala Val Phe Tyr Gln Val Tyr Pro Arg Ser Phe Lys Asp Thr
   50 55 60
   Asn Gly Asp Gly Ile Gly Asp Ile Lys Gly Ile Ile Glu Lys Leu Asp
   65 70 75 80
   Tyr Leu Lys Ala Leu Gly Ile Asp Ala Ile Trp Ile Asn Pro His Tyr
   85 90 95
   Asp Ser Pro Asn Thr Asp Asn Gly Tyr Asp Ile Arg Asp Tyr Arg Lys
   100 105 110
   Ile Met Lys Glu Tyr Gly Thr Met Glu Asp Phe Asp Arg Leu Ile Ser
   115 120 125
   Glu Met Lys Lys Arg Asn Met Arg Leu Met Ile Asp Val Val Ile Asn
   130 135 140
   His Thr Ser Asp Gln Asn Glu Trp Phe Val Lys Ser Lys Ser Ser Lys
   145 150 155 160
   Asp Asn Pro Tyr Arg Gly Tyr Tyr Phe Trp Lys Asp Ala Lys Glu Gly
   165 170 175
   Gln Ala Pro Asn Asn Tyr Pro Ser Phe Phe Gly Gly Ser Ala Trp Gln
               180                 185                 190
   Lys Asp Glu Lys Thr Asn Gln Tyr Tyr Leu His Tyr Phe Ala Lys Gln
           195                 200                 205
   Gln Pro Asp Leu Asn Trp Asp Asn Pro Lys Val Arg Gln Asp Leu Tyr
       210                 215                 220
   Ala Met Leu Arg Phe Trp Leu Asp Lys Gly Val Ser Gly Leu Arg Phe
   225                 230                 235                 240
   Asp Thr Val Ala Thr Tyr Ser Lys Ile Pro Asp Phe Pro Asn Leu Thr
                   245                 250                 255
   Gln Gln Gln Leu Lys Asn Phe Ala Ala Glu Tyr Thr Lys Gly Pro Asn
               260                 265                 270
   Ile His Arg Tyr Val Asn Glu Met Asn Arg Glu Val Leu Ser His Tyr
           275                 280                 285
   Asp Ile Ala Thr Ala Gly Glu Ile Phe Gly Val Pro Leu Asp Gln Ser
       290                 295                 300
   Ile Lys Phe Phe Asp Arg Arg Arg Asp Glu Leu Asn Ile Ala Phe Thr
   305                 310                 315                 320
   Phe Asp Leu Ile Arg Leu Asp Arg Asp Ser Asp Gln Arg Trp Arg Arg
                   325                 330                 335
   Lys Glu Trp Lys Leu Ser Gln Phe Arg Gln Val Ile Asp Asn Val Asp
               340                 345                 350
   Arg Thr Ala Gly Glu Tyr Gly Trp Asn Ala Phe Phe Leu Asp Asn His
           355                 360                 365
   Asp Asn Pro Arg Ala Val Ser His Phe Gly Asp Asp Arg Pro Gln Trp
       370                 375                 380
   Arg Glu Pro Ser Ala Lys Ala Leu Ala Thr Leu Thr Leu Thr Gln Arg
   385                 390                 395                 400
   Ala Thr Pro Phe Ile Tyr Gln Gly Ser Glu Leu Gly Met Thr Asn Tyr
                   405                 410                 415
   Pro Phe Lys Ala Ile Asp Glu Phe Asp Asp Ile Glu Val Lys Gly Phe
               420                 425                 430
   Trp His Asp Tyr Val Glu Thr Gly Lys Val Lys Ala Asp Glu Phe Leu
           435                 440                 445
   Gln Asn Val Arg Leu Thr Ser Arg Asp Asn Ser Arg Thr Pro Phe Gln
       450                 455                 460
   Trp Asp Thr Ser Lys Asn Ala Gly Phe Thr Ser Gly Lys Pro Trp Phe
   465                 470                 475                 480
   Lys Val Asn Pro Asn Tyr Gln Glu Ile Asn Ala Val Ser Gln Val Ala
                   485                 490                 495
   Gln Pro Asp Ser Val Phe Asn Tyr Tyr Arg Gln Leu Ile Lys Ile Arg
               500                 505                 510
   His Asn Ile Pro Ala Leu Thr Tyr Gly Thr Tyr Thr Asp Leu Asp Pro
           515                 520                 525
   Ala Asn Asp Ser Val Tyr Ala Tyr Thr Arg Ser Leu Gly Ala Glu Lys
       530                 535                 540
   Tyr Leu Val Val Val Asn Phe Gln Glu Gln Val Met Arg Tyr Lys Leu
   545                 550                 555                 560
   Pro Asp Asn Leu Ser Ile Glu Lys Val Ile Ile Glu Ser Asn Ser Lys
                   565                 570                 575
   Asn Val Val Lys Lys Asn Asp Ala Leu Leu Glu Leu Lys Pro Trp Gln
               580                 585                 590
   Ser Gly Val Tyr Lys Leu Asn Gln
           595                 600
<210> 8
   <211> 600
   <212> PRT
   <213> Erwinia rhapontici
<400> 8
   Met Ser Ser Gln Gly Leu Lys Thr Ala Val Ala Ile Phe Leu Ala Thr
   1               5                   10                  15
   Thr Phe Ser Ala Thr Ser Tyr Gln Ala Cys Ser Ala Gly Pro Asp Thr
               20                  25                  30
   Ala Pro Ser Leu Thr Val Gln Gln Ser Asn Ala Leu Pro Thr Trp Trp
           35                  40                  45
   Lys Gln Ala Val Phe Tyr Gln Val Tyr Pro Arg Ser Phe Lys Asp Thr
       50                  55                  60
   Asn Gly Asp Gly Ile Gly Asp Leu Asn Gly Ile Ile Glu Asn Leu Asp
   65                  70                  75                  80
   Tyr Leu Lys Lys Leu Gly Ile Asp Ala Ile Trp Ile Asn Pro His Tyr
                   85                  90                  95
   Asp Ser Pro Asn Thr Asp Asn Gly Tyr Asp Ile Arg Asp Tyr Arg Lys
               100                 105                 110
   Ile Met Lys Glu Tyr Gly Thr Met Glu Asp Phe Asp Arg Leu Ile Ser
           115                 120                 125
   Glu Met Lys Lys Arg Asn Met Arg Leu Met Ile Asp Ile Val Ile Asn
       130                 135                 140
   His Thr Ser Asp Gln His Ala Trp Phe Val Gln Ser Lys Ser Gly Lys
   145                 150                 155                 160
   Asn Asn Pro Tyr Arg Asp Tyr Tyr Phe Trp Arg Asp Gly Lys Asp Gly
                   165                 170                 175
   His Ala Pro Asn Asn Tyr Pro Ser Phe Phe Gly Gly Ser Ala Trp Glu
               180                 185                 190
   Lys Asp Asp Lys Ser Gly Gln Tyr Tyr Leu His Tyr Phe Ala Lys Gln
           195                 200                 205
   Gln Pro Asp Leu Asn Trp Asp Asn Pro Lys Val Arg Gln Asp Leu Tyr
       210                 215                 220
   Asp Met Leu Arg Phe Trp Leu Asp Lys Gly Val Ser Gly Leu Arg Phe
   225                 230                 235                 240
   Asp Thr Val Ala Thr Tyr Ser Lys Ile Pro Asn Phe Pro Asp Leu Ser
                   245                 250                 255
   Gln Gln Gln Leu Lys Asn Phe Ala Glu Glu Tyr Thr Lys Gly Pro Lys
               260                 265                 270
   Ile His Asp Tyr Val Asn Glu Met Asn Arg Glu Val Leu Ser His Tyr
           275                 280                 285
   Asp Ile Ala Thr Ala Gly Glu Ile Phe Gly Val Pro Leu Asp Lys Ser
   290 295 300
   Ile Lys Phe Phe Asp Arg Arg Arg Asn Glu Leu Asn Ile Ala Phe Thr
   305 310 315 320
   Phe Asp Leu Ile Arg Leu Asp Arg Asp Ala Asp Glu Arg Trp Arg Arg
   325 330 335
   Lys Asp Trp Thr Leu Ser Gln Phe Arg Lys Ile Val Asp Lys Val Asp
   340 345 350
   Gln Thr Ala Gly Glu Tyr Gly Trp Asn Ala Phe Phe Leu Asp Asn His
   355 360 365
   Asp Asn Pro Arg Ala Val Ser His Phe Gly Asp Asp Arg Pro Gln Trp
   370 375 380
   Arg Glu His Ala Ala Lys Ala Leu Ala Thr Leu Thr Leu Thr Gln Arg
   385 390 395 400
   Ala Thr Pro Phe Ile Tyr Gln Gly Ser Glu Leu Gly Met Thr Asn Tyr
   405 410 415
   Pro Phe Lys Lys Ile Asp Asp Phe Asp Asp Val Glu Val Lys Gly Phe
   420 425 430
   Trp Gln Asp Tyr Val Glu Thr Gly Lys Val Lys Ala Glu Glu Phe Leu
   435 440 445
   Gln Asn Val Arg Gln Thr Ser Arg Asp Asn Ser Arg Thr Pro Phe Gln
   450 455 460
   Trp Asp Ala Ser Lys Asn Ala Gly Phe Thr Ser Gly Thr Pro Trp Leu
   465 470 475 480
   Lys Ile Asn Pro Asn Tyr Lys Glu Ile Asn Ser Ala Asp Gln Ile Asn
   485 490 495
   Asn Pro Asn Ser Val Phe Asn Tyr Tyr Arg Lys Leu Ile Asn Ile Arg
   500 505 510
   His Asp Ile Pro Ala Leu Thr Tyr Gly Ser Tyr Ile Asp Leu Asp Pro
   515 520 525
   Asp Asn Asn Ser Val Tyr Ala Tyr Thr Arg Thr Leu Gly Ala Glu Lys
   530 535 540
   Tyr Leu Val Val Ile Asn Phe Lys Glu Glu Val Met His Tyr Thr Leu
   545                 550                 555                 560
   Pro Gly Asp Leu Ser Ile Asn Lys Val Ile Thr Glu Asn Asn Ser His
                   565                 570                 575
   Thr Ile Val Asn Lys Asn Asp Arg Gln Leu Arg Leu Glu Pro Trp Gln
               580                 585                 590
   Ser Gly Ile Tyr Lys Leu Asn Pro
           595                 600
<210> 9
   <211> 550
   <212> PRT
   <213> Erwinia rhapontici
<400> 9
   Met Arg Ser Thr Pro His Trp Lys Glu Ala Val Val Tyr Gln Val Tyr
   1               5                   10                  15
   Pro Arg Ser Phe Met Asp Ser Asn Gly Asp Gly Thr Gly Asp Leu Asn
               20                  25                  30
   Gly Ile Ile Ser Lys Leu Asp Tyr Leu Gln Gln Leu Gly Ile Thr Leu
           35                  40                  45
   Leu Trp Leu Ser Pro Val Tyr Arg Ser Pro Met Asp Asp Asn Gly Tyr
       50                  55                  60
   Asp Ile Ser Asp Tyr Glu Glu Ile Ala Asp Ile Phe Gly Ser Met Ser
   65                  70                  75                  80
   Asp Met Glu Arg Leu Ile Ala Glu Ala Lys Ala Arg Asp Ile Gly Ile
                   85                  90                  95
   Leu Met Asp Leu Val Val Asn His Thr Ser Asp Glu His Pro Trp Phe
               100                 105                 110
   Ile Asp Ala Leu Ser Ser Lys Asn Ser Ala Tyr Arg Asp Phe Tyr Ile
           115                 120                 125
   Trp Arg Ala Pro Ala Ala Asp Gly Gly Pro Pro Asp Asp Ser Arg Ser
       130                 135                 140
   Asn Phe Gly Gly Ser Ala Trp Thr Leu Asp Glu Ala Ser Gly Glu Tyr
   145                 150                 155                 160
   Tyr Leu His Gln Phe Ser Thr Arg Gln Pro Asp Leu Asn Trp Glu Asn
   165 170 175
   Pro Arg Val Arg Glu Ala Ile His Ala Met Met Asn Arg Trp Leu Asp
   180 185 190
   Lys Gly Ile Gly Gly Phe Arg Met Asp Val Ile Asp Leu Ile Gly Lys
   195 200 205
   Glu Val Asp Pro Gln Ile Met Ala Asn Gly Arg His Pro His Leu Tyr
   210 215 220
   Leu Gln Gln Met Asn Arg Ala Thr Phe Gly Pro Arg Gly Ser Val Thr
   225 230 235 240
   Val Gly Glu Thr Trp Ser Ala Thr Pro Glu Asp Ala Leu Leu Tyr Ser
   245 250 255
   Ala Glu Glu Arg Gln Glu Arg Gln Glu Leu Thr Met Val Phe Gln Phe
   260 265 270
   Glu His Ile Lys Leu Phe Trp Asp Glu Gln Tyr Gly Lys Trp Cys Asn
   275 280 285
   Gln Pro Phe Asp Leu Leu Arg Phe Lys Ala Val Ile Asp Lys Trp Gln
   290 295 300
   Thr Ala Leu Ala Asp His Gly Trp Asn Ser Leu Phe Trp Ser Asn His
   305 310 315 320
   Asp Leu Pro Arg Ala Val Ser Lys Phe Gly Asp Asp Gly Glu Tyr Arg
   325 330 335
   Val Val Ser Ala Lys Met Leu Ala Thr Ala Leu His Cys Leu Lys Gly
   340 345 350
   Thr Pro Tyr Ile Tyr Gln Gly Glu Glu Ile Gly Met Thr Asn Val Asn
   355 360 365
   Phe Ala Asp Ile Asp Asp Tyr Arg Asp Ile Glu Ser Leu Asn Leu Tyr
   370 375 380
   Gln Glu Arg Ile Ala Glu Gly Met Ser His Glu Ala Met Met Arg Gly
   385 390 395 400
   Ile His Ala Asn Gly Pro Asp Asn Ala Arg Thr Pro Met Gln Trp Thr
   405 410 415
   Ala Val His Met Pro Gly Leu Pro Pro Val Ser Pro Gly Leu Arg Leu
               420                 425                 430
   Ile Leu Thr Ser Gly Gln Trp Asn Val Ala Ala Ala Leu Asp Asp Pro
           435                 440                 445
   Asp Ser Val Phe Tyr His Tyr Gln Lys Leu Val Ala Leu Arg Lys Gln
       450                 455                 460
   Leu Pro Leu Leu Val His Gly Asp Phe Arg Gln Ile Val Val Glu His
   465                 470                 475                 480
   Pro Gln Val Phe Ala Trp Leu Arg Thr Leu Gly Glu Gln Thr Leu Val
                   485                 490                 495
   Val Ile Asn Asn Phe Thr Arg Asp Ala Val Met Leu Ala Ile Pro Asp
               500                 505                 510
   Asn Leu Gln Ser Gln Gln Gly Arg Cys Leu Ile Asn Asn Tyr Ala Pro
           515                 520                 525
   Arg Glu Gln Leu Glu Pro Ile Met Glu Leu Gln Pro Tyr Glu Ser Phe
       530                 535                 540
   Ala Leu Leu Ile Glu Arg
   545                 550
<210> 10
   <211> 632
   <212> PRT
   <213> Erwinia rhapontici
<220>
   <221> misc_feature
   <222> (236)..(236)
   <223> Xaa can be any naturally occurring amino acid
<220>
   <221> misc_feature
   <222> (449)..(449)
   <223> Xaa can be any naturally occurring amino acid
<400> 10
   Met Ser Ser Gln Glu Leu Lys Ala Ala Val Ala Ile Phe Leu Ala Thr
   1               5                   10                  15
   Thr Phe Ser Ala Thr Ser Tyr Gln Ala Cys Ser Ala Gly Pro Asp Thr
               20                  25                  30
   Ala Pro Ser Leu Thr Val Gln Gln Ser Asn Ala Leu Pro Thr Trp Trp
           35                  40                  45
   Lys Gln Ala Val Phe Tyr Gln Val Tyr Pro Arg Ser Phe Lys Asp Thr
       50                  55                  60
   Asn Gly Asp Gly Ile Gly Asp Leu Asn Gly Ile Ile Glu Asn Leu Asp
   65                  70                  75                  80
   Tyr Leu Lys Lys Leu Gly Ile Asp Ala Ile Trp Ile Asn Pro His Tyr
                   85                  90                  95
   Asp Ser Pro Asn Thr Asp Asn Gly Tyr Asp Ile Arg Asp Tyr Arg Lys
               100                 105                 110
   Ile Met Lys Glu Tyr Gly Thr Met Glu Asp Phe Asp Arg Leu Ile Ser
           115                 120                 125
   Glu Met Lys Lys Arg Asn Met Arg Leu Met Ile Asp Ile Val Ile Asn
       130                 135                 140
   His Thr Ser Asp Gln His Ala Trp Phe Val Gln Ser Lys Ser Gly Lys
   145                 150                 155                 160
   Asn Asn Pro Tyr Arg Asp Tyr Tyr Phe Trp Arg Asp Gly Lys Asp Gly
                   165                 170                 175
   His Ala Pro Asn Asn Tyr Pro Ser Phe Phe Gly Gly Ser Ala Trp Glu
               180                 185                 190
   Lys Asp Asp Lys Ser Gly Gln Tyr Tyr Leu His Tyr Phe Ala Lys Gln
           195                 200                 205
   Gln Pro Asp Leu Asn Trp Asp Asn Pro Lys Val Arg Gln Asp Leu Tyr
       210                 215                 220
   Asp Met Leu Arg Phe Trp Leu Asp Lys Gly Val Xaa Gly Leu Arg Phe
   225                 230                 235                 240
   Asp Thr Val Ala Thr Tyr Ser Lys Ile Pro Asn Phe Pro Asp Leu Ser
                   245                 250                 255
   Gln Gln Gln Leu Lys Asn Phe Ala Glu Glu Tyr Thr Lys Gly Pro Lys
               260                 265                 270
   Ile His Asp Tyr Val Asn Glu Met Asn Arg Glu Val Leu Ser His Tyr
           275                 280                 285
   Asp Ile Ala Thr Ala Gly Glu Ile Phe Gly Val Pro Leu Asp Lys Ser
       290                 295                 300
   Ile Lys Phe Phe Asp Arg Arg Arg Asn Glu Leu Asn Ile Ala Phe Thr
   305                 310                 315                 320
   Phe Asp Leu Ile Arg Leu Asp Arg Asp Ala Asp Glu Arg Trp Arg Arg
                   325                 330                 335
   Lys Asp Trp Thr Leu Ser Gln Phe Arg Lys Ile Val Asp Lys Val Asp
               340                 345                 350
   Gln Thr Ala Gly Glu Tyr Gly Trp Asn Ala Phe Phe Leu Asp Asn His
           355                 360                 365
   Asp Asn Pro Arg Ala Val Ser His Phe Gly Asp Asp Arg Pro Gln Trp
       370                 375                 380
   Arg Glu His Ala Ala Lys Ala Leu Ala Thr Leu Thr Leu Thr Gln Arg
   385                 390                 395                 400
   Ala Thr Pro Phe Ile Tyr Gln Gly Ser Glu Leu Gly Met Thr Asn Tyr
                   405                 410                 415
   Pro Phe Lys Lys Ile Asp Asp Phe Asp Asp Val Glu Val Lys Gly Phe
               420                 425                 430
   Trp Gln Asp Tyr Val Glu Thr Gly Lys Val Lys Ala Glu Glu Phe Leu
           435                 440                 445
   Xaa Asn Val Arg Gln Thr Ser Arg Asp Asn Ser Arg Thr Pro Phe Gln
       450                 455                 460
   Trp Asp Ala Ser Lys Asn Ala Gly Phe Thr Ser Gly Thr Pro Trp Leu
   465                 470                 475                 480
   Lys Ile Asn Pro Asn Tyr Lys Glu Ile Asn Ser Ala Asp Gln Ile Asn
                   485                 490                 495
   Asn Pro Asn Ser Val Phe Asn Tyr Tyr Arg Lys Leu Ile Asn Ile Arg
               500                 505                 510
   His Asp Ile Pro Ala Leu Thr Tyr Gly Ser Tyr Ile Asp Leu Ala Pro
           515                 520                 525
   Asp Asn Asn Ser Val Tyr Ala Tyr Thr Arg Thr Phe Gly Ala Glu Lys
       530                 535                 540
   Tyr Leu Val Val Ile Asn Phe Lys Glu Glu Val Met His Tyr Thr Leu
   545 550 555 560
   Pro Gly Asp Leu Ser Ile Asn Lys Val Ile Thr Glu Asn Asn Ser His
   565 570 575
   Thr Ile Val Asn Lys Asn Asp Val Glu Asp Pro Arg Gly Ala Thr Ser
   580 585 590
   Val Cys Ser Pro Phe Gln Ala Gln Lys Arg Pro Gly Asp Pro Gly Tyr
   595 600 605
   Ser Ala Ala His Ser Ile Arg Phe Leu Pro Arg Phe Phe Ala Ser Tyr
   610 615 620
   Arg Gly Asp Ile His Ala Phe Lys
   625 630
<210> 11
   <211> 598
   <212> PRT
   <213> Klebsiella sp.
<400> 11
   Met Ser Phe Val Thr Leu Arg Thr Gly Val Ala Val Ala Leu Ser Ser
   1 5 10 15
   Leu Ile Ile Ser Leu Ala Cys Pro Ala Val Ser Ala Ala Pro Ser Leu
   20 25 30
   Asn Gln Asp Ile His Val Gln Lys Glu Ser Glu Tyr Pro Ala Trp Trp
   35 40 45
   Lys Glu Ala Val Phe Tyr Gln Ile Tyr Pro Arg Ser Phe Lys Asp Thr
   50 55 60
   Asn Asp Asp Gly Ile Gly Asp Ile Arg Gly Ile Ile Glu Lys Leu Asp
   65 70 75 80
   Tyr Leu Lys Ser Leu Gly Ile Asp Ala Ile Trp Ile Asn Pro His Tyr
   85 90 95
   Asp Ser Pro Asn Thr Asp Asn Gly Tyr Asp Ile Ser Asn Tyr Arg Gln
   100 105 110
   Ile Met Lys Glu Tyr Gly Thr Met Glu Asp Phe Asp Ser Leu Val Ala
   115 120 125
   Glu Met Lys Lys Arg Asn Met Arg Leu Met Ile Asp Val Val Ile Asn
   130 135 140
   His Thr Ser Asp Gln His Pro Trp Phe Ile Gln Ser Lys Ser Asp Lys
   145 150 155 160
   Asn Asn Pro Tyr Arg Asp Tyr Tyr Phe Trp Arg Asp Gly Lys Asp Asn
   165 170 175
   Gln Pro Pro Asn Asn Tyr Pro Ser Phe Phe Gly Gly Ser Ala Trp Gln
   180 185 190
   Lys Asp Ala Lys Ser Gly Gln Tyr Tyr Leu His Tyr Phe Ala Arg Gln
   195 200 205
   Gln Pro Asp Leu Asn Trp Asp Asn Pro Lys Val Arg Glu Asp Leu Tyr
   210 215 220
   Ala Met Leu Arg Phe Trp Leu Asp Lys Gly Val Ser Gly Met Arg Phe
   225 230 235 240
   Asp Thr Val Ala Thr Tyr Ser Lys Ile Pro Gly Phe Pro Asn Leu Thr
   245 250 255
   Pro Glu Gln Gln Lys Asn Phe Ala Glu Gln Tyr Thr Met Gly Pro Asn
   260 265 270
   Ile His Arg Tyr Ile Gln Glu Met Asn Arg Lys Val Leu Ser Arg Tyr
   275 280 285
   Asp Val Ala Thr Ala Gly Glu Ile Phe Gly Val Pro Leu Asp Arg Ser
   290 295 300
   Ser Gln Phe Phe Asp Arg Arg Arg His Glu Leu Asn Met Ala Phe Met
   305 310 315 320
   Phe Asp Leu Ile Arg Leu Asp Arg Asp Ser Asn Glu Arg Trp Arg His
   325 330 335
   Lys Ser Trp Ser Leu Ser Gln Phe Arg Gln Ile Ile Ser Lys Met Asp
   340 345 350
   Val Thr Val Gly Lys Tyr Gly Trp Asn Thr Phe Phe Leu Asp Asn His
   355 360 365
   Asp Asn Pro Arg Ala Val Ser His Phe Gly Asp Asp Arg Pro Gln Trp
       370                 375                 380
   Arg Glu Ala Ser Ala Lys Ala Leu Ala Thr Ile Thr Leu Thr Gln Arg
   385                 390                 395                 400
   Ala Thr Pro Phe Ile Tyr Gln Gly Ser Glu Leu Gly Met Thr Asn Tyr
                   405                 410                 415
   Pro Phe Arg Gln Leu Asn Glu Phe Asp Asp Ile Glu Val Lys Gly Phe
               420                 425                 430
   Trp Gln Asp Tyr Val Gln Ser Gly Lys Val Thr Ala Thr Glu Phe Leu
           435                 440                 445
   Asp Asn Val Arg Leu Thr Ser Arg Asp Asn Ser Arg Thr Pro Phe Gln
       450                 455                 460
   Trp Asn Asp Thr Leu Asn Ala Gly Phe Thr Arg Gly Lys Pro Trp Phe
   465                 470                 475                 480
   His Ile Asn Pro Asn Tyr Val Glu Ile Asn Ala Glu Arg Glu Glu Thr
                   485                 490                 495
   Arg Glu Asp Ser Val Leu Asn Tyr Tyr Lys Lys Met Ile Gln Leu Arg
               500                 505                 510
   His His Ile Pro Ala Leu Val Tyr Gly Ala Tyr Gln Asp Leu Asn Pro
           515                 520                 525
   Gln Asp Asn Thr Val Tyr Ala Tyr Thr Arg Thr Leu Gly Asn Glu Arg
       530                 535                 540
   Tyr Leu Val Val Val Asn Phe Lys Glu Tyr Pro Val Arg Tyr Thr Leu
   545                 550                 555                 560
   Pro Ala Asn Asp Ala Ile Glu Glu Val Val Ile Asp Thr Gln Gln Gln
                   565                 570                 575
   Ala Ala Ala Pro His Ser Thr Ser Leu Ser Leu Ser Pro Trp Gln Ala
               580                 585                 590
   Gly Val Tyr Lys Leu Arg
           595
<210> 12
   <211> 598
   <212> PRT
   <213> Klebsiella sp. NK33-98-8
<400> 12
   Met Ser Phe Val Thr Leu Arg Thr Gly Val Ala Val Ala Leu Ser Ser
   1               5                   10                  15
   Leu Ile Ile Ser Leu Ala Cys Pro Ala Val Ser Ala Ala Pro Ser Leu
               20                  25                  30
   Asn Gln Asp Ile His Val Gln Lys Glu Ser Glu Tyr Pro Ala Trp Trp
           35                  40                  45
   Lys Glu Ala Val Phe Tyr Gln Ile Tyr Pro Arg Ser Phe Lys Asp Thr
       50                  55                  60
   Asn Asp Asp Gly Ile Gly Asp Ile Arg Gly Ile Ile Glu Lys Leu Asp
   65                  70                  75                  80
   Tyr Leu Lys Ser Leu Gly Ile Asp Ala Ile Trp Ile Asn Pro His Tyr
                   85                  90                  95
   Asp Ser Pro Asn Thr Asp Asn Gly Tyr Asp Ile Ser Asn Tyr Arg Gln
               100                 105                 110
   Ile Met Lys Glu Tyr Gly Thr Met Glu Asp Phe Asp Asn Leu Val Ala
           115                 120                 125
   Glu Met Lys Lys Arg Asn Met Arg Leu Met Ile Asp Val Val Ile Asn
       130                 135                 140
   His Thr Ser Asp Gln His Pro Trp Phe Ile Gln Ser Lys Ser Asp Lys
   145                 150                 155                 160
   Asn Ser Pro Tyr Arg Asn Tyr Tyr Phe Trp Arg Asp Gly Lys Asp Asn
                   165                 170                 175
   Gln Pro Pro Asn Asn Tyr Pro Ser Phe Phe Gly Gly Ser Ala Trp Gln
               180                 185                 190
   Lys Asp Val Lys Ser Gly Gln Tyr Tyr Leu His Tyr Phe Ala Arg Gln
           195                 200                 205
   Gln Pro Asp Leu Asn Trp Asp Asn Pro Lys Val Arg Glu Asp Leu Tyr
       210                 215                 220
   Ala Met Leu Arg Phe Trp Leu Asp Lys Gly Val Ser Gly Met Arg Phe
   225                 230                 235                 240
   Asp Thr Val Ala Thr Tyr Ser Lys Ile Pro Gly Phe Pro Asn Leu Thr
                   245                 250                 255
   Pro Glu Gln Gln Lys Asn Phe Ala Glu Gln Tyr Thr Met Gly Pro Asn
               260                 265                 270
   Ile His Arg Tyr Ile Gln Glu Met Asn Arg Glu Val Leu Ser Arg Tyr
           275                 280                 285
   Asp Val Ala Thr Ala Gly Glu Ile Phe Gly Val Pro Leu Asp Arg Ser
       290                 295                 300
   Ser Gln Phe Phe Asp Arg Arg Arg His Glu Leu Asn Met Ala Phe Met
   305                 310                 315                 320
   Phe Asp Leu Ile Arg Leu Asp Arg Asp Ser Asn Glu Arg Trp Arg His
                   325                 330                 335
   Lys Ser Trp Ser Leu Ser Gln Phe Arg Gln Ile Ile Ser Lys Met Asp
               340                 345                 350
   Val Thr Val Gly Lys Tyr Gly Trp Asn Thr Phe Phe Leu Asp Asn His
           355                 360                 365
   Asp Asn Pro Arg Ala Val Ser His Phe Gly Asp Asp Arg Pro Gln Trp
       370                 375                 380
   Arg Glu Ala Ser Ala Lys Ala Leu Ala Thr Ile Thr Leu Thr Gln Arg
   385                 390                 395                 400
   Ala Thr Pro Phe Ile Tyr Gln Gly Ser Glu Leu Gly Met Thr Asn Tyr
                   405                 410                 415
   Pro Phe Arg Gln Leu Asn Glu Phe Asp Asp Ile Glu Val Lys Gly Phe
               420                 425                 430
   Trp Gln Asp Tyr Val Gln Ser Gly Lys Val Thr Ala Thr Glu Phe Leu
           435                 440                 445
   Asp Asn Val Arg Leu Thr Ser Arg Asp Asn Ser Arg Thr Pro Phe Gln
       450                 455                 460
   Trp Asn Asp Thr Leu Asn Ala Gly Phe Thr Arg Gly Lys Pro Trp Phe
   465                 470                 475                 480
   His Ile Asn Pro Asn Tyr Val Glu Ile Asn Ala Glu Arg Glu Glu Thr
                   485                 490                 495
   Arg Glu Asp Ser Val Leu Asn Tyr Tyr Lys Lys Met Ile Gln Leu Arg
                   500                 505                 510
   His His Ile Pro Ala Leu Val Tyr Gly Ala Tyr Gln Asp Leu Asn Pro
               515                 520                 525
   Gln Asp Asn Thr Val Tyr Ala Tyr Thr Arg Thr Leu Gly Asn Glu Arg
       530                 535                 540
   Tyr Leu Val Val Val Asn Phe Glu Glu Tyr Pro Val Arg Tyr Thr Leu
   545                 550                 555                 560
   Pro Ala Asn Asp Ala Ile Glu Glu Val Val Ile Asp Thr Gln Gln Gln
                   565                 570                 575
   Ala Thr Ala Pro His Ser Thr Ser Leu Ser Leu Ser Pro Trp Gln Ala
               580                 585                 590
   Gly Val Tyr Lys Leu Arg
           595
<210> 13
   <211> 598
   <212> PRT
   <213> Klebsiella planticola
<400> 13
   Met Ser Phe Ile Thr Leu Arg Thr Gly Val Ala Val Ala Leu Ser Ser
   1               5                   10                  15
   Leu Met Ile Ser Gln Ala Cys Pro Ala Val Ser Ala Ala Pro Ser Val
               20                  25                  30
   Asn Gln Asn Ile His Val His Lys Glu Ser Glu Tyr Pro Ala Trp Trp
           35                  40                  45
   Lys Glu Ala Val Phe Tyr Gln Ile Tyr Pro Arg Ser Phe Lys Asp Thr
       50                  55                  60
   Asn Asp Asp Gly Ile Gly Asp Ile Arg Gly Ile Ile Glu Lys Leu Asp
   65                  70                  75                  80
   Tyr Leu Lys Ser Leu Gly Ile Asp Ala Ile Trp Ile Asn Pro His Tyr
                   85                  90                  95
   Asp Ser Pro Asn Thr Asp Asn Gly Tyr Asp Ile Ser Asn Tyr Arg Gln
               100                 105                 110
   Ile Met Lys Glu Tyr Gly Thr Met Glu Asp Phe Asp Asn Leu Val Ala
   115 120 125
   Glu Met Lys Lys Arg Asn Met Arg Leu Met Ile Asp Val Val Ile Asn
   130 135 140
   His Thr Ser Asp Gln His Pro Trp Phe Ile Gln Ser Lys Ser Asp Lys
   145 150 155 160
   Asn Asn Pro Tyr Arg Asp Tyr Tyr Phe Trp Arg Asp Gly Lys Asp Asn
   165 170 175
   Gln Pro Pro Asn Asn Tyr Pro Ser Phe Phe Gly Gly Ser Ala Trp Gln
   180 185 190
   Lys Asp Ala Lys Ser Gly Gln Tyr Tyr Leu His Tyr Phe Ala Arg Gln
   195 200 205
   Gln Pro Asp Leu Asn Trp Asp Asn Pro Lys Val Arg Glu Asp Leu Tyr
   210 215 220
   Ala Met Leu Arg Phe Trp Leu Asp Lys Gly Val Ser Ser Met Arg Phe
   225 230 235 240
   Asp Thr Val Ala Thr Tyr Ser Lys Ile Pro Gly Phe Pro Asn Leu Thr
   245 250 255
   Pro Glu Gln Gln Lys Asn Phe Ala Glu Gln Tyr Thr Met Gly Pro Asn
   260 265 270
   Ile His Arg Tyr Ile Gln Glu Met Asn Arg Lys Val Leu Ser Arg Tyr
   275 280 285
   Asp Val Ala Thr Ala Gly Glu Ile Phe Gly Val Pro Leu Asp Arg Ser
   290 295 300
   Ser Gln Phe Phe Asp Pro Arg Arg His Glu Leu Asn Met Ala Phe Met
   305 310 315 320
   Phe Asp Leu Ile Arg Leu Asp Arg Asp Ser Asn Glu Arg Trp Arg His
   325 330 335
   Lys Ser Trp Ser Leu Ser Gln Phe Arg Gln Ile Ile Ser Lys Met Asp
   340 345 350
   Val Thr Val Gly Lys Tyr Gly Trp Asn Thr Phe Phe Leu Asp Asn His
   355 360 365
   Asp Asn Pro Arg Ala Val Ser His Phe Gly Asp Asp Arg Pro Gln Trp
       370                 375                 380
   Arg Glu Ala Ser Ala Lys Ala Leu Ala Thr Ile Thr Leu Thr Gln Arg
   385                 390                 395                 400
   Ala Thr Pro Phe Ile Tyr Gln Gly Ser Glu Leu Gly Met Thr Asn Tyr
                   405                 410                 415
   Pro Phe Arg Gln Leu Asn Glu Phe Asp Asp Ile Glu Val Lys Gly Phe
               420                 425                 430
   Trp Gln Asp Tyr Val Gln Ser Gly Lys Val Thr Ala Thr Glu Phe Leu
           435                 440                 445
   Asp Asn Val Arg Leu Thr Ser Arg Asp Asn Ser Arg Thr Pro Phe Gln
       450                 455                 460
   Trp Asn Asp Thr Leu Asn Ala Gly Phe Thr Arg Gly Lys Pro Trp Phe
   465                 470                 475                 480
   His Ile Asn Pro Asn Tyr Val Glu Ile Asn Ala Glu Arg Glu Glu Thr
                   485                 490                 495
   Arg Glu Asp Ser Val Leu Asn Tyr Tyr Lys Lys Met Ile Gln Leu Arg
               500                 505                 510
   His His Ile Pro Ala Leu Val Tyr Gly Ala Tyr Gln Asp Leu Asn Pro
           515                 520                 525
   Gln Asp Asn Thr Val Tyr Ala Tyr Thr Arg Thr Leu Gly Asn Glu Arg
       530                 535                 540
   Tyr Leu Val Val Val Asn Phe Lys Glu Tyr Pro Val Arg Tyr Thr Leu
   545                 550                 555                 560
   Pro Ala Asn Asp Ala Ile Glu Glu Val Val Ile Asp Thr Gln Gln Gln
                   565                 570                 575
   Ala Thr Ala Pro His Ser Thr Ser Leu Ser Leu Ser Pro Trp Gln Ala
               580                 585                 590
   Gly Val Tyr Lys Leu Arg
           595
<210> 14
   <211> 599
   <212> PRT
   <213> Erwinia sp. Ejp617
<400> 14
   Met Ser Arg Phe Thr Leu Ser Thr Val Met Ala Leu Leu Val Ala Pro
   1               5                   10                  15
   Val Leu Ser Val Leu Pro Gly Gln Val Met Ala Gly Lys Met Asp Met
               20                  25                  30
   Ala Thr Thr Gln Leu Ala Gln Lys Ser Asp Asp Phe Pro Ala Trp Trp
           35                  40                  45
   Lys Gln Ala Val Phe Tyr Gln Val Tyr Pro Arg Ser Phe Lys Asp Thr
       50                  55                  60
   Asn Gly Asp Gly Ile Gly Asp Leu Lys Gly Ile Ile Glu Lys Leu Asp
   65                  70                  75                  80
   Tyr Leu Lys Arg Leu Gly Val Asp Ala Ile Trp Ile Asn Pro His Tyr
                   85                  90                  95
   Asp Ser Pro Asn Thr Asp Asn Gly Tyr Asp Ile Arg Asp Tyr Arg Lys
               100                 105                 110
   Ile Met Lys Glu Tyr Gly Thr Met Glu Asp Phe Asp Arg Leu Ile Ala
           115                 120                 125
   Glu Met Asn Lys Arg Asn Met Arg Leu Met Ile Asp Ile Val Ile Asn
       130                 135                 140
   His Thr Ser Asp Gln His Ser Trp Phe Val Gln Ser Lys Gly Ser Lys
   145                 150                 155                 160
   Asp Asn Pro Tyr Arg Asp Tyr Tyr Phe Trp Arg Asp Gly Lys Asn Gly
                   165                 170                 175
   Gln Pro Pro Asn Asn Tyr Pro Ser Phe Phe Gly Gly Ser Ala Trp Lys
               180                 185                 190
   Lys Glu Asp Asn Ser Gly Gln Tyr Tyr Leu His Tyr Phe Ala Thr Gln
           195                 200                 205
   Gln Pro Asp Leu Asn Trp Asp Asn Pro Lys Val Arg Glu Asp Leu Tyr
       210                 215                 220
   Ala Met Leu Arg Phe Trp Leu Asp Lys Gly Val Ala Gly Leu Arg Phe
   225 230 235 240
   Asp Thr Val Ala Thr Tyr Ala Lys Ile Pro Gly Phe Pro Asp Leu Thr
   245 250 255
   Pro Gln Gln Arg Lys Asn Phe Ala Arg Thr Tyr Thr Glu Gly Pro Ser
   260 265 270
   Ile His Arg Tyr Ile Lys Glu Met His Gln Gln Val Phe Ser His Tyr
   275 280 285
   Asn Ile Ala Thr Ala Gly Glu Ile Phe Gly Val Pro Leu Glu Lys Ser
   290 295 300
   Ile Asp Tyr Phe Asp Arg Arg Arg Gly Glu Leu Asn Ile Ala Phe Thr
   305 310 315 320
   Phe Asp Leu Ile Arg Leu Asp Arg Gly Val Glu Glu Arg Trp Arg Gln
   325 330 335
   Lys Ala Trp Ser Leu Thr Asp Phe Arg Gln Thr Ile Asp Lys Val Asp
   340 345 350
   Arg Val Ala Gly Lys Tyr Gly Trp Asn Ala Phe Phe Leu Asp Asn His
   355 360 365
   Asp Asn Pro Arg Ala Val Ser His Phe Gly Asp Asp Arg Pro Gln Trp
   370 375 380
   Arg Gln Ala Ser Ala Lys Ala Leu Ala Thr Leu Met Ile Thr Gln Arg
   385 390 395 400
   Ala Thr Pro Phe Ile Tyr Gln Gly Ser Glu Leu Gly Met Thr Asn Tyr
   405 410 415
   Pro Phe Lys Ser Ile Ala Asp Phe Asp Asp Ile Glu Val Lys Gly Phe
   420 425 430
   Trp Gln Asp Tyr Val Ser Ser Gly Lys Val Asp Pro Glu Glu Phe Met
   435 440 445
   Arg Asn Val Arg Leu Thr Ser Arg Asp Asn Ser Arg Thr Pro Phe Gln
   450 455 460
   Trp Asp Glu Ser Ala Asn Ala Gly Phe Thr Ser Gly Thr Pro Trp Phe
   465 470 475 480
   Asn Val Asn Pro Asn Tyr Lys Leu Ile Asn Ala Ala Asp Gln Thr Arg
   485 490 495
   Asp Pro Asp Ser Val Phe Asn Tyr Tyr Arg Lys Leu Ile Gly Leu Arg
   500 505 510
   His Ala Ile Pro Ala Leu Thr Tyr Gly Glu Tyr Lys Asp Leu Asp Pro
   515 520 525
   Asn Asn Asp Thr Val Tyr Ala Phe Thr Arg Thr His Gly Asp Lys Arg
   530 535 540
   Tyr Leu Val Val Ile Asn Phe Lys Glu Asn Val Val Asn Tyr Arg Leu
   545 550 555 560
   Pro Asp Gln Leu Thr Ile Arg Gln Thr Leu Ser Glu Ser Ser Ala Ile
   565 570 575
   Gln Pro Val Ala Glu Asn Ala Arg Glu Leu Leu Leu Gln Pro Trp Gln
   580 585 590
   Ser Gly Ile Tyr Gln Leu Asn
   595
<210> 15
   <211> 600
   <212> PRT
   <213> Azotobacter vinelandii
<400> 15
   Met Gln Thr Ala Ser Phe Arg Arg Thr Leu Ser Ser Val Met Leu Gly
   1 5 10 15
   Ala Leu Ala Ile Cys Gln Thr Ala Met Gly Thr Pro Asn Thr Gly Ser
   20 25 30
   Asp Thr Ala Arg Glu Asp Val Ala Lys Thr Pro Ala Ala Arg Pro Asp
   35 40 45
   Ala Pro Trp Trp Lys Glu Ala Val Val Tyr Gln Val Tyr Pro Arg Ser
   50 55 60
   Phe Asn Asp Thr Asn Gly Asp Gly Ile Gly Asp Leu Asn Gly Ile Thr
   65 70 75 80
   Ala Lys Leu Asp Tyr Leu Lys Ala Leu Gly Val Asp Thr Ile Trp Leu
   85 90 95
   Ser Pro His Phe Asp Ser Pro Asn Ala Asp Asn Gly Tyr Asp Ile Arg
               100                 105                 110
   Asp Tyr Arg Lys Val Met Ser Glu Phe Gly Asp Met Asp Asp Phe Glu
           115                 120                 125
   Arg Leu Leu Ala Gly Met Asn Lys Arg Gly Met Arg Leu Ile Ile Asp
       130                 135                 140
   Leu Val Val Asn His Ser Ser Asp Glu His Arg Trp Phe Val Glu Ser
   145                 150                 155                 160
   Arg Arg Ser Lys Asp Asn Pro Tyr Arg Asp Tyr Tyr Thr Trp Arg Asp
                   165                 170                 175
   Gly Lys Asp Gly Ala Ala Pro Asn Asn Tyr Pro Ser Phe Phe Gly Gly
               180                 185                 190
   Ser Ala Trp Lys Lys Asp Glu Ala Thr Gly Gln Tyr Tyr Leu His Tyr
           195                 200                 205
   Phe Ala Gly Lys Gln Pro Asp Leu Asn Trp Glu Asn Pro Glu Val Arg
       210                 215                 220
   Ala Glu Val His Asp Ile Met Arg Phe Trp Leu Asp Lys Gly Val Ser
   225                 230                 235                 240
   Gly Phe Arg Met Asp Val Ile Pro Phe Ile Ser Lys Gln Asp Gly Leu
                   245                 250                 255
   Pro Asp Leu Pro Ala Gln Ala Leu Ala His Pro Glu Phe Val Tyr Ala
               260                 265                 270
   Asn Gly Pro Arg Ile His Glu Tyr Leu Gln Glu Met Asn Arg Glu Val
           275                 280                 285
   Leu Ser Arg Tyr Asp Thr Met Thr Val Gly Glu Ala Phe Gly Ile Thr
       290                 295                 300
   Phe Glu Gln Ala Pro Leu Phe Thr Asp Ala Arg Arg His Glu Leu Asn
   305                 310                 315                 320
   Met Ile Phe His Phe Asp Leu Val Arg Leu Asp Arg Asp Gly Trp Arg
                   325                 330                 335
   Lys Lys Asp Trp Thr Leu Pro Glu Leu Lys Ala Thr Tyr Ala Arg Ile
               340                 345                 350
   Asp Arg Thr Gly Gly Asp His Gly Trp Asn Thr Ser Phe Leu Gly Asn
           355                 360                 365
   His Asp Asn Pro Arg Ala Val Ser His Phe Gly Asp Asp Ser Pro Glu
       370                 375                 380
   Trp Arg Ala Ala Ser Ala Lys Ala Leu Ala Thr Met Met Leu Thr Gln
   385                 390                 395                 400
   Arg Ala Thr Pro Phe Leu Tyr Gln Gly Asp Glu Leu Gly Met Thr Asn
                   405                 410                 415
   Tyr Pro Phe Arg Gly Leu Glu Asp Tyr Asp Asp Val Glu Val Lys Gly
               420                 425                 430
   Gln Trp Arg Asp Phe Val Glu Ser Gly Lys Val Ser Ala Asp Glu Tyr
           435                 440                 445
   Leu Ala His Leu Arg Gln Thr Ser Arg Asp Asn Ala Arg Thr Pro Met
       450                 455                 460
   Gln Trp Ser Asp Ala Pro Asn Gly Gly Phe Thr Thr Gly Lys Pro Trp
   465                 470                 475                 480
   Leu Ala Val Asn Pro Asn Tyr Pro Gln Val Asn Ala Ala Ser Gln Val
                   485                 490                 495
   Asp Asp Pro Gly Ser Ile Tyr His His Tyr Arg Arg Leu Leu Glu Val
               500                 505                 510
   Arg Arg Gln Thr Pro Ala Leu Ile His Gly Gln Phe Arg Asp Leu Asp
           515                 520                 525
   Pro Ala Asn Pro Lys Val Phe Ala Tyr Thr Arg Thr Leu Asp Asp Lys
       530                 535                 540
   Arg Tyr Leu Val Leu Ile Asn Phe Thr Arg Glu Thr Val Ala Tyr Asp
   545                 550                 555                 560
   Leu Pro Glu Gly Leu Lys Ile Ala Ala Thr Leu Leu Asp Asn Gly Ala
                   565                 570                 575
   Ala Gln Glu Ser Met Gln Pro Gly Ala Ala Ser Val Thr Leu Gln Pro
               580                 585                 590
   Trp Gln Ala Thr Ile Tyr Arg Leu
           595                 600
<210> 16
   <211> 599
   <212> PRT
   <213> Enterobacter sp.
<400> 16
   Met Ser Phe Phe Lys Lys Leu Thr Gly Val Ala Val Thr Phe Ser Ser
   1               5                   10                  15
   Leu Phe Met Thr Leu Ala Pro Ala Ala Tyr Ser Ala Glu Thr Ser Val
               20                  25                  30
   Thr Gln Ser Ile Gln Thr Gln Lys Glu Ser Thr Leu Pro Ala Trp Trp
           35                  40                  45
   Lys Glu Ala Val Phe Tyr Gln Ile Tyr Pro Arg Ser Phe Lys Asp Thr
       50                  55                  60
   Asn Gly Asp Gly Ile Gly Asp Ile Arg Gly Ile Ile Glu Lys Leu Asp
   65                  70                  75                  80
   Tyr Leu Lys Ser Leu Gly Ile Asp Ala Ile Trp Ile Asn Pro His Tyr
                   85                  90                  95
   Asp Ser Pro Asn Thr Asp Asn Gly Tyr Asp Ile Arg Asp Tyr Glu Lys
               100                 105                 110
   Ile Met Gln Glu Tyr Gly Thr Met Glu Asp Phe Asp Thr Leu Val Ser
           115                 120                 125
   Glu Met Lys Lys Arg Asn Met Arg Leu Met Ile Asp Val Val Ile Asn
       130                 135                 140
   His Thr Ser Asp Gln His Pro Trp Phe Ile Gln Ser Lys Ser Ser Lys
   145                 150                 155                 160
   Glu Asn Pro Tyr Arg Glu Tyr Tyr Phe Trp Arg Asp Gly Lys Asp Asn
                   165                 170                 175
   Gln Pro Pro Asn Asn Tyr Pro Ser Phe Phe Gly Gly Ser Ala Trp Gln
               180                 185                 190
   Lys Asp Asp Lys Thr Gly Gln Tyr Tyr Leu His Tyr Phe Ala Arg Gln
           195                 200                 205
   Gln Pro Asp Leu Asn Trp Asp Asn Pro Lys Val Arg Gly Asp Leu Tyr
       210                 215                 220
   Ala Met Leu Arg Phe Trp Leu Asp Lys Gly Val Ser Gly Met Arg Phe
   225 230 235 240
   Asp Thr Val Ala Thr Tyr Ser Lys Ile Pro Gly Phe Pro Asp Leu Thr
   245 250 255
   Pro Glu Gln Gln Lys Asn Phe Ala Glu Gln Tyr Thr Thr Gly Pro Asn
   260 265 270
   Ile His Arg Tyr Leu Gln Glu Met Lys Gln Glu Val Leu Ser Arg Tyr
   275 280 285
   Asp Val Val Thr Ala Gly Glu Ile Phe Gly Val Pro Leu Glu Arg Ser
   290 295 300
   Ser Asp Phe Phe Asp Arg Arg Arg Asn Glu Leu Asp Met Ser Phe Met
   305 310 315 320
   Phe Asp Leu Ile Arg Leu Asp Arg Asp Ser Asn Glu Arg Trp Arg His
   325 330 335
   Lys Lys Trp Thr Leu Ser Gln Phe Arg Gln Ile Ile Asn Lys Met Asp
   340 345 350
   Ser Asn Ala Gly Glu Tyr Gly Trp Asn Thr Phe Phe Leu Asp Asn His
   355 360 365
   Asp Asn Pro Arg Ala Val Ser His Phe Gly Asp Asp Ser Pro Gln Trp
   370 375 380
   Ile Glu Pro Ser Ala Lys Ala Leu Ala Thr Ile Ile Leu Thr Gln Arg
   385 390 395 400
   Ala Thr Pro Phe Ile Phe Gln Gly Ser Glu Leu Gly Met Thr Asn Tyr
   405 410 415
   Pro Phe Lys Lys Leu Asn Glu Phe Asp Asp Ile Glu Val Lys Gly Phe
   420 425 430
   Trp Gln Asp Tyr Val Gln Thr Gly Lys Val Ser Ala Glu Glu Phe Ile
   435 440 445
   Asp Asn Val Arg Leu Thr Ser Arg Asp Asn Ser Arg Thr Pro Phe Gln
   450 455 460
   Trp Asn Asp Arg Lys Asn Ala Gly Phe Thr Ser Gly Lys Pro Trp Phe
   465                 470                 475                 480
   Arg Ile Asn Pro Asn Tyr Val Glu Ile Asn Ala Asp Lys Glu Leu Ile
                   485                 490                 495
   Arg Asn Asp Ser Val Leu Asn Tyr Tyr Lys Glu Met Ile Lys Leu Arg
               500                 505                 510
   His Lys Thr Pro Ala Leu Ile Tyr Gly Thr Tyr Lys Asp Ile Ser Pro
           515                 520                 525
   Glu Asp Asp Ser Val Tyr Ala Tyr Thr Arg Thr Leu Gly Lys Glu Arg
       530                 535                 540
   Tyr Leu Val Val Ile Asn Phe Thr Glu Lys Thr Val Arg Tyr Pro Leu
   545                 550                 555                 560
   Pro Glu Asn Asn Val Ile Lys Ser Ile Leu Ile Glu Ala Asn Gln Asn
                   565                 570                 575
   Lys Thr Ala Glu Lys Gln Ser Thr Val Leu Thr Leu Ser Pro Trp Gln
               580                 585                 590
   Ala Gly Val Tyr Glu Leu Gln
           595

## Beschreibung

### Gebiet der Erfindung

Gegenstand der Erfindung ist ein Verfahren zur Isomerisierung eines saccharosehaltigen Pflanzensaftes zu Isomaltulose.

### Stand der Technik

Die enzymatische Isomerisierung von Saccharose in wässriger Lösung mit Hilfe von Isomaltulose Synthasen (Saccharose Glucosylmutasen, EC 5.4.99.11) zu Isomaltulose ist ein bekannter Prozess.

So beschreibt DE1049800 ein Verfahren zur fermentativen Herstellung von Isomaltulose aus saccharosehaltigen Pflanzensäften mittels des Bakterienstammes *Protaminobacter rubrum.* Alternativ kann auch eine enzymatische Umwandlung mit toten Organismen stattfinden. Die Zellmasse wird chargenweise durch Zentrifugation am Ende der Umsetzung abgetrennt und das Produkt weiter aufgearbeitet. Dieses Verfahren findet sich in der Literatur bei H. Schiweck, alimenta 19, 5-16, 1980 wieder.

EP0001099 beschreibt ein Verfahren zur kontinuierlichen Fermentation der Bakterienstämme *Protaminobacter rubrum* (CBS574.77) und *Serratia plymuthica* (ATCC 15928) unter gleichzeitiger Isomerisierung der Saccharose zu Isomaltulose mit unter anderem Dick- oder Dünnsaft als Substrat, wobei die Substratlösung selber Nährmedium zum Wachstum der Zellen darstellt. Die Zellen werden nach der Umsetzung mittels Zentrifugation von der Produktlösung abgetrennt. Eine von der eigentlichen Isomerisierung separate Anzucht der biokatalytisch wirksamen Organismen in einem anderen Medium als dem Substratmedium wird ausdrücklich als nachteilig herausgestellt.

DE3241788 beschreibt ein Verfahren zur Herstellung von 1-O-α-D-Glucopyranosido-D-Fructose aus wässrigen Saccharoselösungen mit freien Zellen der Bakterienstämme aus der Gruppe *Protaminobacter rubrum* (CBD574.77), *Serratia plymuthica* (ATCC 15928), *Serratia marescens* (NCIB 8285), *Leuconostoc mesenteroides* (NRRL-B-512 F) und *Erwinia rhapontici* (NCPPB 1578). In dem beschriebenen Verfahren kann Dicksaft verwendet werden, und dieser dient ebenfalls als die Kohlenstoffquelle für den Wachstumsprozess der Zellen. Im Falle von immobilisierten Zellen wird auf reine Saccharoselösung zurückgegriffen.

Im Fall der in den beiden vorgenannten Schriften offenbarten fermentativen Herstellung von Isomaltulose besteht die Möglichkeit des Einsatzes von wirtschaftlich und energetisch günstigem Dicksaft unter sterilen Bedingungen. Allerdings muss hier der Nachteil erwähnt werden, dass ein Teil der Saccharose zur Bildung von Zellmasse genutzt wird, sowie aufwendige sterile Hardware als auch eine aufwendige Abtrennung der Zellsuspension vom Produktstrom notwendig ist.

EP0983374 beschreibt ein Verfahren zur gleichzeitigen Herstellung von Isomaltulose und Betain mit immobilisierten Bakterienstämmen aus der Gruppe *Protaminobacter rubrum* (CBD574.77), *Serratia plymuthica* (ATCC 15928) und *Erwinia rhapontici* (NCPPB 1578). Das Herstellverfahren geht von Melasse aus, einer Zusammensetzung, die am Ende des Zuckerherstellverfahrens anfällt. Der Einsatz von Dicksaft als Substrat ist in diesem Verfahren nicht möglich und bedarf einer expliziten Abreicherung der Saccharose durch zum Beispiel Kristallisation.

WO97/44478 beschreibt ein Verfahren zur Herstellung von Isomaltulose mit lebenden, immobilisierten Bakterienstämmen aus der Gruppe *Protaminobacter rubrum* (CBD574.77), *Serratia plymuthica* (ATCC 15928) und *Erwinia rhapontici* (ATCC 29284). Das Herstellverfahren geht von Melasse oder reiner Saccharoselösung aus.

EP0028900 beschreibt ein Verfahren zur Herstellung von Isomaltulose mit immobilisierten Zellen des Bakterienstammes *Erwinia rhapontici* (NCPPB 1578). Das Herstellverfahren geht aus von einer reinen Saccharoselösung mit einer maximalen Beimischung von 15 %v/v Melasse.

DE2217628, EP49472, EP3038219 und EP91063 beschreiben Verfahren mit immobilisierten Bakterienzellen zur enzymatischen Umwandlung von reiner Saccharose zu Isomaltulose. EP0625578 setzt hierzu Bakterienstämme aus der Gruppe von *Protaminobacter rubrum* (CBS 574.77), *Serratia plymuthica* (ATCC 15928), *Serratia marcescens* (NCIB 8285), *Leuconostoc mesenteroides* (NRRL-B 512 F (ATCC 1083 a)) und *Erwinia rhapontici* (NCPPB 1578) ein. EP0392556 und EP1257638 beschreiben den Einsatz von Bakterienstämmen aus der Gruppe von *Klebsiella terrigena* JCM 1687, *Klebsiella* sp. No. 88 (FERM BP-2838) und *Klebsiella singaporensis* LX3 and LX21.

Isomerisationsverfahren mit immobilisierten Zellen werden in DE3133123 und EP0915986 beschrieben und bedienen sich im Rahmen der Immobilisierungsverfahren der Enzymkatalysatoren des Calciumalginats oder der Ionentauscher.

Allen bekannten Verfahren zur Isomerisierung von Saccharose zu Isomaltulose mit immobilisierten Zellen ist gemein, dass nur die Endprodukte des Zuckerherstellprozesses genutzt werden, nämlich reine Saccharose oder Melasse.

US 5,578,339 offenbart ein Verfahren zur Herstellung von Isomaltulose mittels immobilisierter Bakterien des Stammes CBS 574.77 von Protaminobacter rubrum. Dabei wurde als Ausgangssubstrat eine Saccharose Lösung von 35-45 % verwendet (wässrige Lösung, kein Dicksaft).

Aufgabe der vorliegenden Erfindung war es daher, ein Verfahren bereitzustellen, welches es ermöglicht, Isomaltulose aus einer gut verfügbaren Saccharose-Quelle zu gewinnen.

### Beschreibung der Erfindung

Überraschenderweise wurde nun gefunden, dass der Einsatz von saccharosehaltigen Pflanzensäften für die Herstellung von Isomaltulose unter Einsatz von immobilisierten Enzymkomplexen diese Aufgabe zu lösen vermag.
Gegenstand der vorliegenden Erfindung ist daher ein Verfahren zur Herstellung von Isomaltulose umfassend die Verfahrensschritte
A) in Kontakt Bringen eines immobilisierten Enzymkomplexes, welches die Umsetzung von Saccharose zu Isomaltulose zu katalysieren vermag, mit einer saccharosehaltigen Lösung;
B) Isomerisierung mindestens eines Teiles der Saccharose zu Isomaltulose;
C) Abtrennen des Enzymkomplexes und gegebenenfalls
D) weiteres Aufreinigen der Isomaltulose,
dadurch gekennzeichnet, dass als saccharosehaltige Lösung Dicksaft, enthaltend mehr als 20 Gew.-% Saccharose bezogen auf den gesamten Saft eingesetzt wird.

Die Erfindung sieht dabei in vorteilhafter Weise die Verwendung von saccharosehaltigen Pflanzensäften als Substrat für die Isomerisierung vor.
Überraschenderweise wird durch die vorliegende Erfindung trotz der Tatsache, dass es sich bei den eingesetzten saccharosehaltigen Lösungen um ungereinigte und somit hochkomplexe Substratmischungen handelt, dieselbe Ausbeute und gar eine höhere Selektivität in der Isomerisierung wie mit hochreiner Saccharose erzielt.
Ein Vorteil der vorliegenden Erfindung liegt darin, dass durch die erhöhte Standzeit der immobilisierten Zellen in Kombination mit einem rohen Pflanzensaft als Substrat eine wesentlich höhere Ressourceneffizienz erreicht wird, da der Aufreinigungsschritt zur hochreinen Saccharose eingespart wird.
Ein weiterer Vorteil der vorliegenden Erfindung ist es, dass durch den direkten Einsatz von Dicksaft Energie und Betriebsmittel eingespart werden können.
Noch ein Vorteil der vorliegenden Erfindung ist es, dass durch den Einsatz des Dicksaftes keinerlei Pufferlösungen oder andere Zusätze zur Stabilisierung der enzymatischen Aktivität notwendig sind, sondern eine natürliche Stabilisierung der eingesetzten Enzyme stattfindet.

Noch ein weiterer Vorteil der vorliegenden Erfindung ist es, dass ein Ausbluten des Bettes im Gegensatz zum Einsatz von rein wässriger Saccharoselösung durch den Einsatz von saccharosehaltigen Pflanzensäften verhindert wird.
Noch ein Vorteil der vorliegenden Erfindung ist es, dass durch den Einsatz des Dicksaftes während der Isomerisierung der Gehalt an den kariogenen Monosacchariden Fructose und Glucose verglichen zu dem Einsatz von gereinigter Saccharoselösung verringert werden kann.

Unter dem Begriff "Isomaltulose" ist 6-O-α-D-Glucopyranosido-D-Fructose zu verstehen.
Unter dem Begriff "Enzymkomplex" ist eine mindestens ein aktives Enzym aufweisende Zusammensetzung oder Mischungszusammensetzung zu verstehen, die auch komplexer Natur sein kann, wie beispielsweise eine lebende Zelle. Weitere Beispiele eines Enzymkomplexes sind Fusionsproteine, in denen das mindestens eine aktive Enzym mit mindestens einem weiteren Polypeptid verknüpft ist, aber auch ein aufgereinigtes Enzym selber kann einen Enzymkomplex im Sinne der vorliegenden Erfindung darstellen.
Unter dem Begriff "immobilisierter Enzymkomplex" ist im Zusammenhang mit der vorliegenden Erfindung ein Enzymkomplex zu verstehen, der an eine Matrix gebunden oder von einer Matrix eingeschlossen ist, so dass der Enzymkomplex in seiner freien Diffusion oder in seiner freien Bewegung in einer wässrigen Lösung eingeschränkt, beispielsweise verlangsamt, ist.
Unter dem Begriff "Dünnsaft" ist im Zusammenhang mit der vorliegenden Erfindung das Produkt zu verstehen, welches bei der Zuckerherstellung aus Zuckerrüben oder Zuckerrohr nach der Saftreinigung vorliegt. Dünnsaft enthält typischerweise 10-20 Gew.-% Saccharose bezogen auf den gesamten Saft.
Unter dem Begriff "Dicksaft" ist im Zusammenhang mit der vorliegenden Erfindung der eingedickte "Dünnsaft" zu verstehen, welcher mehr als 20 Gew.-% Saccharose bezogen auf den gesamten Saft enthält.
Alle angegebenen Prozent (%) sind wenn nicht anders angegeben Massenprozent.

In dem erfindungsgemäßen Verfahren wird als saccharosehaltige Lösung vorteilhaft Dicksaft eingesetzt; dieser kann gegebenenfalls bis zu einem Volumenverhältnis von 1:5, bezogen auf Dicksaft zu Wasser, mit Wasser verdünnt werden, wodurch eine besser kontrollierbare Isomerisierung erhalten wird.
Insbesondere wird die Verdünnung des Dicksaftes mit Wasser so gewählt, dass am Anfang des Verfahrensschrittes A) im Dicksaft eine Saccharosekonzentration von 20-80 Gew.-%, insbesondere von 30-50 Gew.-% bezogen auf den gesamten Saft vorliegt.

Insbesondere Dicksaft von Zuckerrüben ist vorteilhaft einzusetzen.
Das in dem immobilisierten Enzymkomplex enthaltene Enzym ist vorzugsweise mindestens eine Saccharose Glucosylmutase der Enzymklasse EC 5.4.99.11. Besonders bevorzugt werden Saccharose Glucosylmutasen aus *Protaminobacter rubrum,* insbesondere der Stamm *Protaminobacter rubrum* CBS 574.77 und solche mit Seq ID Nr. 5 und 6; *Protaminobacter ruber* Z12; *Serratia plymuthica,* insbesondere der Stamm *Serratia* plymuthica ATCC 15928; *Serratia odorifera,* insbesondere der Stamm *Serratia odorifera* 4Rx13 (Seq ID Nr. 7); *Serratia marcescens,* insbesondere der Stamm *Serratia marcescens* NCIB 8285; *Leuconostoc mesenteroides,* insbesondere der Stamm *Leuconostoc mesenteroides* ATCC 1083 a; *Erwinia rhapontici,* insbesondere die Stamm *Erwinia rhapontici* ATCC29283 (Seq ID Nr. 1), NCPPB 1578, DSM 4484 (Seq ID Nr. 8), NX-5 (Seq ID Nr. 9) und WAC2928 (Seq ID Nr. 10); *Erwinia* sp., insbesondere der Stamm *Erwinia* sp. D12; *Agrobacterium radiobacter,* insbesondere der Stamm *Agrobacterium radiobacter* MX-232; *Klebsiella terrigena,* insbesondere der Stamm *Klebsiella terrigena* JCM 1687; *Klebsiella* sp. insbesondere die Stämme *Klebsiella* sp. FERM BP-2838, LX3 (Seq ID Nr. 11) und NK33-98-8 (Seq ID Nr. 12); *Klebsiella pneumoniae,* insbesondere der Stamm *Klebsiella pneumoniae* 342 (Seq ID Nr. 4); *Klebsiella singaporensis,* insbesondere der Stamm *Klebsiella singaporensis* LX21; *Pseudomonas mesoacidophila,* insbesondere der Stamm *Pseudomonas mesoacidophila* MX-45 (Seq ID Nr. 2); *Pantoea dispersa,* insbesondere der Stamm *Pantoea dispersa* UQ68J (Seq ID NR. 3); *Klebsiella planticola,* insbesondere die Stämme *Klebsiella planticola* CCRC 19112, MX10 und UQ14S (Seq ID NR. 13); *Enterobacter* sp., insbesondere der Stamm *Enterobacter* sp. FMB-1 (Seq ID NR. 16), SZ62 und Ejp617 (Seq ID NR. 14) ; Azotobacter vinelandii DJ (Seq ID NR. 15) in dem erfindungsgemäßen Verfahren eingesetzt, wobei *Protaminobacter rubrum* CBS 574.77 und *Protaminobacter ruber* Z12 besonders bevorzugt sind.

Die Enzyme können in aufgereinigter Form als Polypeptide eingesetzt werden. Zur erleichterten Aufreinigung können diese als Fusionsproteine vorliegen, wobei beispielsweise ein, die leichte Aufreinigung ermöglichender *tag,* wie beispielsweise ein His-*tag*, ein Strep-tag, ein GST-*tag* oder ein MBP-*tag*, an das Enzym fusioniert ist.

Es ist in dem erfindungsgemäßen Verfahren bevorzugt, dass es sich bei dem Enzymkomplex um ganze Zellen handelt, welche bevorzugt ausgewählt sind aus der Gruppe *Protaminobacter rubrum,* insbesondere der Stamm *Protaminobacter rubrum* CBS 574.77; *Protaminobacter ruber* Z12; *Serratia plymuthica,* insbesondere der Stamm *Serratia* plymuthica ATCC 15928; *Serratia odorifera,* insbesondere der Stamm *Serratia odorifera* 4Rx13; *Serratia marcescens,* insbesondere der Stamm *Serratia marcescens* NCIB 8285; *Leuconostoc mesenteroides,* insbesondere der Stamm *Leuconostoc mesenteroides* ATCC 1083 a; *Erwinia rhapontici,* insbesondere die Stämm *Erwinia rhapontici* ATCC29283, NCPPB 1578, DSM 4484, NX-5 und WAC2928; *Erwinia* sp., insbesondere der Stamm *Erwinia* sp. D12; *Agrobacterium radiobacter,* insbesondere der Stamm *Agrobacterium radiobacter* MX-232; *Klebsiella terrigena,* insbesondere der Stamm *Klebsiella terrigena* JCM 1687; *Klebsiella* sp. insbesondere die Stämme *Klebsiella* sp. FERM BP-2838, LX3 und NK33-98-8; *Klebsiella pneumoniae,* insbesondere der Stamm *Klebsiella pneumoniae* 342; *Klebsiella singaporensis,* insbesondere der Stamm *Klebsiella singaporensis* LX21; *Pseudomonas mesoacidophila,* insbesondere der Stamm *Pseudomonas mesoacidophila* MX-45; *Pantoea dispersa,* insbesondere der Stamm *Pantoea dispersa* UQ68J; *Klebsiella planticola,* insbesondere die Stämme *Klebsiella planticola* CCRC 19112, MX10 und UQ14S; *Enterobacter* sp., insbesondere der Stamm *Enterobacter* sp. FMB-1, SZ62 und Ejp617; *Azotobacter vinelandii DJ,* wobei *Protaminobacter rubrum* CBS 574.77 und *Protaminobacter ruber Z12* besonders bevorzugt sind.

Die Immobilisierung der Enzymkomplexe kann beispielsweise in Form von CLEAs (*insoluble crosslinked enzyme aggregates*) (Cao, L. et al., 2000, Cross-linked enzyme aggregates: a simple and effective method for the immobilization of penicillin acylase, Org. Lett., 2: 1361-1264) oder an festen Trägermaterialien natürlichen oder synthetischen Ursprungs erfolgen. Natürliche Materialien sind z.B. Polysaccharide wie Alginat, Agarose, Sepharose, Cellulose und dessen Derivate (z. B. DEAE- oder CM-Cellulose). Es können auch modifizierte Sepharosen, wie z.B. durch Epoxy-aktivierte-, Bromcyan-aktivierte-, NHS-aktivierte-, Thiol-aktivierte Sepharose eingesetzt werden. Diese Sepharosen sind beispielsweise bei den Firmen GE Healthcare, BioRad, Sigma und Pierce kommerziell erhältlich.

Als synthetische organische Polymere können Polystyrene-Derivate, Polyacrylate, insbesondere epoxidaktivierte Acrylharzperlen (Eupergit), Polymethacrylate, Polyacrylamide, Vinyl- und Allylpolymere, Polyester oder Polyamide eingesetzt werden.
Als anorganische Träger sind Materialien auf der Basis von Silicium- oder Aluminiumoxiden bzw. Gemischen daraus möglich.
Die Immobilisierung der Enzymkomplexe kann auch durch Verkapselung in polymeren porösen Gelen z.B. hydrophoben Sol-Gel Materialien aus RSi(OCH₃)₃ bzw. Gemischen aus RSi(OCH₃)₃ und Si(OCH₃)₄ (Reetz, M.T.; Zonta, A.; Simpelkamp, J.; Rufinska, A.; Tesche, B. J. Sol-Gel Sci. Technol. 1996, 7, p. 35-43) oder aus porösen polymeren Silica-Gelen erfolgen (Elgren, T.M.; Zadvorny, O.A.; Brecht, E.; Douglas, T.; Zorin, N.A.; Maroney, M. J. & Peters, J. W.).

Neben einer Immobilisierung ist außerdem eine Mehrfachverwendung des Enzyms in Enzymmembranreaktoren denkbar.

Die in dem erfindungsgemäßen Verfahren eingesetzten Enzymkomplexe, insbesondere Zellen, werden bevorzugt in Polysacchariden wie Alginat, Pectin, Carrageenan, Chitosan oder Polyvinylalcoholen, wie beispielsweise Lentikats, oder Mischungen daraus, insbesondere in Alginat immobilisiert; vgl hierzu beispielsweise Shimizu, H., et al. (1997) Screening of novel microbial enzymes for the production of biologically and chemically useful compounds, in: Advances in biochemical engineering biotechnology, Vol58: New Enzymes.for Organic Synthesis (Scheper, T., ed.) pp. 45-88, Springer, New York.

Eine besonders bevorzugt einzusetzende Immobilisierung für jegliche Form der in dem erfindungsgemäßen Verfahren eingesetzten Enzymkomplexe stellt das in der EP2011865 beschriebene Verfahren dar, bei dem die auf einem inerten Träger immobilisierten Enzymkomplexe mit einer durch Hydrosilylierung gewonnenen Siliconbeschichtung versehen werden.

Es ist erfindungsgemäß bevorzugt, dass in Verfahrensschritt B) ein pH-Wert von 4 bis 9,5 vorliegt. Dieser pH-Wert wird vorteilhaft mit Hilfe einer Säure, insbesondere einer anorganischen Säure, bevorzugt aus der Gruppe Schwefelsäure, Salzsäure oder Essigsäure, eingestellt.
Es ist weiterhin erfindungsgemäß bevorzugt, dass in Verfahrensschritt B) eine Temperatur von 20 bis 40°C, bevorzugt von 25 bis 35°C, gemessen in der saccharosehaltigen Lösung vorliegt. In Verfahrensschritt C) wird der Enzymkomplex von der Isomaltulose abgetrennt; dies erfolgt beispielsweise durch Filtration, Sedimentation oder Zentrifugation. Aufgrund des Immobilisatcharakters des Enzymkomplexes ist diese Abtrennung gegenüber nicht immobilisiertem Enzym erleichtert.
Aus Gründen der Verfahrensökonomie ist es erfindungsgemäß bevorzugt, wenn der immobilisierte Enzymkomplex in Form eines Festbettes eingesetzt wird, durch welches die saccharosehaltige Lösung strömt (H. Schiweck, Zuckerind. (1990), 115 (7), 555-565).

Entsprechende Verfahren im Festbett werden in A. Liese, et. al. Processes in A. Liese, K. Seelbach, C. Wandrey (Eds.), Industrial Biotransformations 2nd Edition (2006), Wiley-VCH, Weinheim, beschrieben. In solch einem Verfahren finden Verfahrenschritte A) bis C) kontinuierlich, und damit ineinander übergehend bis hin zu gleichzeitig, statt.

In dem optionalen Verfahrensschritt D) kann die Isomaltulose weiter aufgereinigt werden. Dies kann insbesondere durch beispielsweise Kristallisation oder Chromatographie erfolgen. Entsprechende Verfahren werden in DE3241788 und EP0983374 beschrieben.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung des Biokatalysators

Von einer Abimpfung des Stammes *Protaminobacter rubrum* (CBS574.77) wurden Zellen mit 1-5 mL eines sterilen Nährmediums bestehend aus 50 g/kg Saccharose , 15 g/kg Maisquellwasser, 7 g/kg Ammoniumsulfat, 0,5 g/kg Hefeextrakt, 1 g/kg Kaliumdihydrogensulfat, 0,41 g/kg Magnesiumchlorid Heptahydrat, 0,004 g/kg Manganchlorid Tetrahydrat, 0,047 g/kg Eisencitrat Monohydrat und 926 g/kg Wasser, bei Bedarf auf pH 7,2 eingestellt, abgeschwemmt. Diese Suspension diente als Impfgut für die Vorkultur, welche 200 mL, obiger Nährlösung, in einem 1-L Schüttelkolben enthält.

Nach einer 20 stündigen Kultivierung bei 30 °C wurde mit der Vorkultur ein 2-L Fermenter, mit einem Liter sterilem Produktionsmedium bestehend aus 50 g/kg Saccharose , 15 g/kg Maisquellwasser, 3 g/kg Ammoniumsulfat, 4 g/kg Ammoniumhydrogenphosphat, 0,5 g/kg Hefeextrakt, 1 g/kg Kaliumdihydrogensulfat, 0,41 g/kg Magnesiumchlorid Heptahydrat, 0,004 g/kg Manganchlorid Tetrahydrat, 0,047 g/kg Eisencitrat Monohydrat und 926 g/kg Wasser, eingestellt auf pH 7,2, in der Art beimpft, dass die initiale optische Dichte (OD₆₀₀) 1 betrug.

Es wurde bei 30 °C, pH 7 (geregelt) und einem pO₂ von 30 % (Kaskade Rührer, Airflow) fermentiert. Saccharose wurde während der Fermentation zugefüttert. Nach 15-20 h war der Fermentationsprozess beendet und die Biomasse konnte für die Immobilisierung geerntet werden.

Hierzu wurde die erhaltene Suspension mit Wasser und einer 4%-igen Alginatlösung im Volumenverhältnis 1:1 vermischt. Diese Suspension wurde dann durch Eintropfen in eine 2%-ige CaCl₂-Lösung immobilisiert. Die erhaltenen Kugeln wurden mit Polyethlyenimin und Glutaraldehyd nachgehärtet. Der erhaltene Biokatalysator ist mehrere Wochen bei 4-10 °C lagerfähig.

### Beispiel 2: Herstellung von Isomaltulose durch Isomerisierung von reiner Saccharoselösung (nicht erfindungsgemäß)

Es wurde aus einem Dicksaft aus Zuckerrüben reine Saccharose wie in Sugarin Ullman's Encyclopedia of Industrial Chemisty (2007*)* beschrieben hergestellt; die Ausbeute an Saccharose beträgt 88,3 %.

Die in Beispiel 1 erhaltenen immobilisierten Zellen wurden in einen temperierbaren Säulenreaktor gefüllt und auf 20-35 °C temperiert. Aus der reinen Saccharose und Leitungswasser wurde eine Saccharoselösung mit 35 bis 45 % Trockensubstanz(TS)-Gehalt hergestellt. Der Säulenreaktor wurde kontinuierlich mit dieser Lösung durchströmt.

Die HPLC-Analyse der aus dem Säulenreaktor austretenden Isomaltuloselösung ergab in diesem Verfahrensschritt mittlere Werte für Ausbeute, Selektivität und Umsatz von:

| | |
|---|---|
| Ausbeute | 85% |
| Selektivität | 91% |
| Umsatz | 94% |

Die Ausbeute dieses Verfahrens bezogen auf eingesetzte Saccharose betrug 75 %.

### Beispiel 3: Herstellung von Isomaltulose durch Isomerisierung von Dicksaft (erfindungsgemäß)

Die Beispiel 1 erhaltenen immobilisierten Zellen werden in einen temperierbaren Säulenreaktor gefüllt und auf 20-35 °C temperiert und mit dem oben beschriebenen, verdünnten Dicksaft aus Zuckerrüben mit einem Gehalt von 35 bis 45 % TS-Saccharose kontinuierlich durchströmt.

Die HPLC-Analyse der aus dem Säulenreaktor austretenden Isomaltuloselösung ergab mittlere Werte für Ausbeute, Selektivität und Umsatz von:

| | |
|---|---|
| Ausbeute | 82% |
| Selektivität | 91% |
| Umsatz | 90% |

Die Ausbeute dieses Verfahrens bezogen auf eingesetzte Saccharose betrug 82 % und liegt somit 7 % über der Ausbeute eines Verfahrens nach dem Stande der Technik.

### Beispiel 4: Selektivitätsvergleich der Isomerisierung von Dicksaft im Vergleich zur Isomerisierung von gereinigter Saccharoselösung

Nach in Beispiel 1 erhaltene, immobilisierte Zellen wurden in einen auf 30 °C temperierten Säulenreaktoren beschickt.

Säulenreaktor 1 wurde mit gereinigter Saccharoselösung kontinuierlich durchströmt, deren Konzentration mit Leitungswasser auf 40 % Trockensubstanz-Gehalt und mit konzentrierter Schwefelsäure auf einen pH-Wert von 6 eingestellt wurde.

Säulenreaktor 2 wurde mit auf 40 % Trockensubstanz-Gehalt an Saccharose verdünntem und mit konzentrierter Schwefelsäure auf einen pH-Wert von 6 eingestellten Dicksaft aus Zuckerrüben kontinuierlich durchströmt.

In beiden Säulenreaktoren wurden die Ausbeuten an Isomaltulose zur besseren Bestimmmbarkeit der Selektivitäten durch Anpassung des Volumenstroms auf einen identischen Wert von 79 % bezogen auf den jeweiligen Trockensubstanz-Gehalt an Saccharose eingestellt. Die analytische Bestimmung erfolgte durch HPLC-Analyse der erhaltenen Isomaltuloselösung.

Die erzielten Selektivitäten waren:

| | |
|---|---|
| Selektivität (Dicksaft) | 85 % |
| Selektivität (Saccharoselösung) | 83 % |

Zusätzlich konnte die Bildung der unerwünschten, da kariogenen, Monosaccharide Fructose und Glucose durch den Einsatz von Dicksaft verglichen zu dem Einsatz von gereinigter Saccharose-Lösung um ca. 10 % (bezogen auf Trockensubstanz) im Endprodukt reduziert werden.

## Patentansprüche

1. Verfahren zur Herstellung von Isomaltulose umfassend die Verfahrensschritte
A) in Kontakt Bringen eines immobiliserten Enzymkomplexes, welches die Umsetzung von Saccharose zu Isomaltulose zu katalysieren vermag, mit einer saccharosehaltigen Lösung;
B) Isomerisierung mindestens eines Teiles der Saccharose zu Isomaltulose;
C) Abtrennen des Enzymkomplexes und gegebenenfalls
D) weiteres Aufreinigen der Isomaltulose,
**dadurch gekennzeichnet,**
**dass** als saccharosehaltige Lösung Dicksaft enthaltend mehr als 20 Gew.-% Saccharose bezogen auf den gesamten Saft eingesetzt wird.

2. Verfahren nach Anspruch 1,
**dadurch gekennzeichnet,**
**dass** als saccharosehaltige Lösung ein mit Wasser verdünnter Dicksaft mit einer Saccharosekonzentration von 20-80 Gew.-%, insbesondere von 30-50 Gew.-% bezogen auf den gesamten Saft eingesetzt wird.

3. Verfahren nach Anspruch 1 oder 2,
**dadurch gekennzeichnet,**
**dass** das in dem immobilisiertem Enzymkomplex enthaltene Enzym mindestens eine Saccharose Glucosylmutase der Enzymklasse EC 5.4.99.11, insbesondere eine aus *Protaminobacter rubrum,* insbesondere der Stamm *Protaminobacter rubrum* CBS 574.77 und solche mit Seq ID Nr. 5 und 6; *Protaminobacter ruber* Z12; *Serratia plymuthica,* insbesondere der Stamm *Serratia* plymuthica ATCC 15928; *Serratia odorifera,* insbesondere der Stamm *Serratia odorifera* 4Rx13 (Seq ID Nr. 7); *Serratia marcescens,* insbesondere der Stamm *Serratia marcescens* NCIB 8285; *Leuconostoc mesenteroides,* insbesondere der Stamm *Leuconostoc mesenteroides* ATCC 1083 a; *Erwinia rhapontici,* insbesondere die Stamm *Erwinia rhapontici* ATCC29283 (Seq ID Nr. 1), NCPPB 1578, DSM 4484 (Seq ID Nr. 8), NX-5 (Seq ID Nr. 9) und WAC2928 (Seq ID Nr. 10); *Erwinia* sp., insbesondere der Stamm *Enwinia* sp. D12; *Agrobacterium radiobacter,* insbesondere der Stamm *Agrobacterium radiobacter* MX-232; *Klebsiella terrigena,* insbesondere der Stamm *Klebsiella terrigena* JCM 1687; *Klebsiella* sp. insbesondere die Stämme *Klebsiella* sp. FERM BP-2838, LX3 (Seq ID Nr. 11) und NK33-98-8 (Seq ID Nr. 12); *Klebsiella pneumoniae,* insbesondere der Stamm *Klebsiella pneumoniae* 342 (Seq ID Nr. 4); *Klebsiella singaporensis,* insbesondere der Stamm *Klebsiella singaporensis* LX21; *Pseudomonas mesoacidophila,* insbesondere der Stamm *Pseudomonas mesoacidophila* MX-45 (Seq ID Nr. 2); *Pantoea dispersa,* insbesondere der Stamm *Pantoea dispersa* UQ68J (Seq ID NR. 3); *Klebsiella planticola,* insbesondere die Stämme *Klebsiella planticola* CCRC 19112, MX10 und UQ14S (Seq ID NR. 13); *Enterobacter* sp., insbesondere der *Enterobacter* sp. FMB-1 (Seq ID NR. 16), SZ62 und Ejp617 (Seq ID NR. 14) ; Azotobacter vinelandii DJ (Seq ID NR. 15) ist.

4. Verfahren nach mindestens einem der vorgenannten Ansprüche,
**dadurch gekennzeichnet,**
**dass** es sich bei dem Enzymkomplex um Zellen, insbesondere um Zellen, handelt, welche ausgewählt sind aus *Protaminobacter rubrum,* insbesondere der Stamm *Protaminobacter rubrum* CBS 574.77; *Protaminobacter ruber* Z12; *Serratia plymuthica,* insbesondere der Stamm *Serratia* plymuthica ATCC 15928; *Serratia odorifera,* insbesondere der Stamm *Serratia odorifera* 4Rx13; *Serratia marcescens,* insbesondere der Stamm *Serratia marcescens* NCIB 8285; *Leuconostoc mesenteroides,* insbesondere der Stamm *Leuconostoc mesenteroides* ATCC 1083 a; *Erwinia rhapontici,* insbesondere die Stämm *Erwinia rhapontici* ATCC29283, NCPPB 1578, DSM 4484, NX-5 und WAC2928; *Erwinia* sp., insbesondere der Stamm *Erwinia* sp. D12; *Agrobacterium radiobacter,* insbesondere der Stamm *Agrobacterium radiobacter* MX-232; *Klebsiella terrigena,* insbesondere der Stamm *Klebsiella terrigena* JCM 1687; *Klebsiella* sp. insbesondere die Stämme *Klebsiella* sp. FERM BP-2838, LX3 und NK33-98-8; *Klebsiella pneumoniae,* insbesondere der Stamm *Klebsiella pneumoniae* 342; *Klebsiella singaporensis,* insbesondere der Stamm *Klebsiella singaporensis* LX21; *Pseudomonas mesoacidophila,* insbesondere der Stamm *Pseudomonas mesoacidophila* MX-45; *Pantoea dispersa,* insbesondere der Stamm *Pantoea dispersa* UQ68J; *Klebsiella planticola,* insbesondere die Stämme *Klebsiella planticola* CCRC 19112, MX10 und UQ14S; *Enterobacter* sp. FMB-1, SZ62 und Ejp617; *Azotobacter vinelandii DJ.*

5. Verfahren nach Anspruch 4,
**dadurch gekennzeichnet,**
**dass** die Zellen in Polysacchariden wie Alginat, Pectin, Carrageenan, Chitosan oder Polyvinylalcoholen, wie beispielsweise Lentikats, oder Mischungen daraus, insbesondere in Alginat immobilisiert sind.

6. Verfahren nach mindestens einem der vorgenannten Ansprüche,
**dadurch gekennzeichnet,**
**dass** in Verfahrensschritt B) ein pH-Wert von 4 bis 9,5 vorliegt.

7. Verfahren nach mindestens einem der vorgenannten Ansprüche,
**dadurch gekennzeichnet,**
**dass** in Verfahrensschritt B) eine Temperatur von 20 bis 40°C vorliegt.

8. Verfahren nach mindestens einem der vorgenannten Ansprüche,
**dadurch gekennzeichnet,**
**dass** der immobilisierte Enzymkomplex in Form eines Festbettes eingesetzt wird, durch welches die saccharosehaltige Lösung strömt.

## Claims

1. Process for preparing isomaltulose, comprising the process steps of
A) contacting an immobilized enzyme complex capable of catalyzing the conversion of sucrose to isomaltulose with a sucrose-containing solution;
B) isomerizing at least some of the sucrose to isomaltulose;
C) removing the enzyme complex, and optionally
D) further purifying the isomaltulose,
**characterized in that**
the sucrose-containing solution used is thick juice containing more than 20% by weight of sucrose based on total juice.

2. Process according to Claim 1,
**characterized in that**
the sucrose-containing solution used is a thick juice diluted with water and having a sucrose concentration of 20-80% by weight, in particular 30-50% by weight, based on total juice.

3. Process according to Claim 1 or 2,
**characterized in that**
the enzyme present in the immobilized enzyme complex is at least one sucrose glucosylmutase of enzyme class EC 5.4.99.11, in particular one from *Protaminobacter rubrum,* in particular the strain *Protaminobacter rubrum* CBS 574.77 and those with Seq ID Nos. 5 and 6; *Protaminobacter ruber* Z12; *Serratia plymuthica,* in particular the strain *Serratia* plymuthica ATCC 15928; *Serratia odorifera,* in particular the strain *Serratia odorifera* 4Rx13 (Seq ID No. 7); *Serratia marcescens,* in particular the strain *Serratia marcescens* NCIB 8285; *Leuconostoc mesenteroides,* in particular the strain *Leuconostoc mesenteroides* ATCC 1083 a; *Erwinia rhapontici,* in particular the strains *Erwinia rhapontici* ATCC29283 (Seq ID No. 1), NCPPB 1578, DSM 4484 (Seq ID No. 8), NX-5 (Seq ID No. 9) and WAC2928 (Seq ID No. 10); *Erwinia* sp., in particular the strain *Erwinia* sp. D12; *Agrobacterium radiobacter,* in particular the strain *Agrobacterium radiobacter* MX-232; *Klebsiella terrigena,* in particular the strain *Klebsiella terrigena* JCM 1687; *Klebsiella* sp., in particular the strains *Klebsiella* sp. FERM BP-2838, LX3 (Seq ID No. 11) and NK33-98-8 (Seq ID No. 12); *Klebsiella pneumoniae,* in particular the strain *Klebsiella pneumoniae* 342 (Seq ID No. 4); *Klebsiella singaporensis,* in particular the strain *Klebsiella singaporensis* LX21; *Pseudomonas mesoacidophila,* in particular the strain *Pseudomonas mesoacidophila* MX-45 (Seq ID No. 2); *Pantoea dispersa,* in particular the strain *Pantoea dispersa* UQ68J (Seq ID NO. 3); *Klebsiella planticola,* in particular the strains *Klebsiella planticola* CCRC 19112, MX10 and UQ14S (Seq ID NO. 13); *Enterobacter* sp., in particular the *Enterobacter* sp. FMB-1 (Seq ID NO. 16), SZ62 and Ejp617 (Seq ID NO. 14); Azotobacter vinelandii DJ (Seq ID NO. 15).

4. Process according to at least one of the preceding claims,
**characterized in that**
the enzyme complex is cells, in particular cells which are selected from *Protaminobacter rubrum,* in particular the strain *Protaminobacter rubrum* CBS 574.77; *Protaminobacter ruber* Z12; *Serratia plymuthica,* in particular the strain *Serratia* plymuthica ATCC 15928; *Serratia odorifera,* in particular the strain *Serratia odorifera* 4Rx13; *Serratia marcescens,* in particular the strain *Serratia marcescens* NCIB 8285; *Leuconostoc mesenteroides,* in particular the strain *Leuconostoc mesenteroides* ATCC 1083 a; *Erwinia rhapontici,* in particular the strains *Erwinia rhapontici* ATCC29283, NCPPB 1578, DSM 4484, NX-5 and WAC2928; *Erwinia* sp., in particular the strain *Erwinia* sp. D12; *Agrobacterium radiobacter,* in particular the strain *Agrobacterium radiobacter* MX-232; *Klebsiella terrigena,* in particular the strain *Klebsiella terrigena* JCM 1687; *Klebsiella* sp., in particular the strains *Klebsiella* sp. FERM BP-2838, LX3 and NK33-98-8; *Klebsiella pneumoniae,* in particular the strain *Klebsiella pneumoniae* 342; *Klebsiella singaporensis,* in particular the strain *Klebsiella singaporensis* LX21; *Pseudomonas mesoacidophila,* in particular the strain *Pseudomonas mesoacidophila* MX-45; *Pantoea dispersa,* in particular the strain *Pantoea dispersa* UQ68J; *Klebsiella planticola,* in particular the strains *Klebsiella planticola* CCRC 19112, MX10 and UQ14S; *Enterobacter* sp. FMB-1, SZ62 and Ejp617; *Azotobacter vinelandii DJ.*

5. Process according to Claim 4,
**characterized in that**
the cells are immobilized in polysaccharides such as alginate, pectin, carrageenan, chitosan, or polyvinyl alcohols, such as lentikats for example, or mixtures thereof, in particular in alginate.

6. Process according to at least one of the preceding claims,
**characterized in that**
a pH of from 4 to 9.5 is present in process step B).

7. Process according to at least one of the preceding claims,
**characterized in that**
the temperature in process step B) is from 20 to 40°C.

8. Process according to at least one of the preceding claims,
**characterized in that**
the immobilized enzyme complex is used in the form of a solid bed through which the sucrose-containing solution flows.

## Revendications

1. Procédé de fabrication d'isomaltulose, comprenant les étapes suivantes :
A) mise en contact d'un complexe enzymatique immobilisé, qui est à même de catalyser la conversion du saccharose en isomaltulose, avec une solution contenant du saccharose ;
B) isomérisation d'au moins une partie du saccharose en isomaltulose ;
C) séparation du complexe enzymatique, et, éventuellement,
D) purification plus poussée de l'isomaltulose, **caractérisé en ce qu'**on utilise en tant que solution contenant du saccharose un sirop contenant plus de 20 % en poids de saccharose, par rapport à la totalité du sirop.

2. Procédé selon la revendication 1, **caractérisé en ce qu'**on utilise en tant que solution contenant du saccharose un sirop dilué à l'eau, ayant une concentration du saccharose de 20 à 80 % en poids, en particulier de 30 à 50 % en poids, par rapport à la totalité du sirop.

3. Procédé selon la revendication 1 ou 2, **caractérisé en ce que** l'enzyme contenue dans le complexe enzymatique immobilisé est une saccharose glucosylmutase de la classe enzymatique EC 5.4.99.11, en particulier une enzyme de *Protaminobacter rubrum,* en particulier la souche *Protaminobacter rubrum* CBS 574.77, et celles ayant les séquences SEQ ID NO:5 et 6 ; *Protaminobacter ruber* Z12 ; *Serratia plymuthica,* en particulier la souche *Serratia plymuthica* ATCC 15928 ; *Serratia odorifera,* en particulier la souche *Serratia odorifera* 4Rx13 (SEQ ID NO:7) ; *Serratia marcescens,* en particulier la souche *Serratia marcescens* NCIB 8285 ; *Leuconostoc mesenteroides,* en particulier la souche *Leuconostoc mesenteroides* ATCC 1083 a ; *Erwinia rhapontici,* en particulier les souches *Erwinia rhapontici* ATCC 29283 (SEQ ID NO:1), NCPPB 1578, DSM 4484 (SEQ ID NO:8), NX-5 (SEQ ID NO:9) et WAC2928 (SEQ ID NO:10) ; *Erwinia* sp., en particulier la souche *Erwinia* sp. D12 ; *Agrobacterium radiobacter,* en particulier la souche *Agrobacterium radiobacter* MX-232 ; *Klebsiella terrigena,* en particulier la souche *Klebsiella terrigena* JCM 1687 ; *Klebsiella* sp., en particulier les souches *Klebsiella* sp. FERM BP-2838, LX3 (SEQ ID NO:11) et NK33-98-8 (SEQ ID NO:12) ; *Klebsiella pneumoniae,* en particulier la souche *Klebsiella pneumoniae* 342 (SEQ ID NO:4) ; *Klebsiella singaporensis,* en particulier la souche *Klebsiella singaporensis* LX21 ; *Pseudomonas mesoacidophila,* en particulier la souche *Pseudomonas mesoacidophila* MX-45 (SEQ ID NO:2) ; *Pantoea dispersa,* en particulier la souche *Pantoea dispersa* UQ68J (SEQ ID NO:3) ; *Klebsiella planticola,* en particulier les souches *Klebsiella planticola* CCRC 19112, MX10 et UQ14S (SEQ ID NO:13) ; *Enterobacter* sp., en particulier *Enterobacter* sp. FMB-1 (SEQ ID NO: 16), SZ62 et Ejp617 (SEQ ID NO:14) ; *Azotobacter vinelandii* DJ (SEQ ID NO:15).

4. Procédé selon au moins l'une des revendications précédentes, **caractérisé en ce que**, pour ce qui concerne le complexe enzymatique, il s'agit de cellules, en particulier de cellules qui sont choisies parmi *Protaminobacter rubrum,* en particulier la souche *Protaminobacter rubrum* CBS 574.77 ; *Protaminobacter ruber* Z12 ; *Serratia plymuthica,* en particulier la souche *Serratia plymuthica* ATCC 15928 ; *Serratia odorifera,* en particulier la souche *Serratia odorifera* 4Rx13 ; *Serratia marcescens,* en particulier la souche *Serratia marcescens* NCIB 8285 ; *Leuconostoc mesenteroides,* en particulier la souche *Leuconostoc mesenteroides* ATCC 1083 a ; *Erwinia rhapontici,* en particulier les souches *Erwinia rhapontici* ATCC 29283, NCPPB 1578, DSM 4484, NX-5 et WAC2928 ; *Erwinia* sp., en particulier la souche *Erwinia* sp. D12 ; *Agrobacterium radiobacter,* en particulier la souche *Agrobacterium radiobacter* MX-232 ; *Klebsiella terrigena,* en particulier la souche *Klebsiella terrigena* JCM 1687 ; *Klebsiella* sp., en particulier les souches *Klebsiella* sp. FERM BP-2838, LX3 et NK33-98-8 ; *Klebsiella pneumoniae,* en particulier la souche *Klebsiella pneumoniae* 342 ; *Klebsiella singaporensis,* en particulier la souche *Klebsiella singaporensis* LX21 ; *Pseudomonas mesoacidophila,* en particulier la souche *Pseudomonas mesoacidophila* MX-45 ; *Pantoea dispersa,* en particulier la souche *Pantoea dispersa* UQ68J ; *Klebsiella planticola,* en particulier les souches *Klebsiella planticola* CCRC 19112, MX10 et UQ14S ; *Enterobacter* sp. FMB-1, SZ62 et Ejp617 ; *Azotobacter vinelandii* DJ.

5. Procédé selon la revendication 4, **caractérisé en ce que** les cellules sont immobilisées dans des polysaccharides tels que l'alginate, la pectine, le carragénane, le chitosan ou les poly(alcools vinyliques), tels que par exemple les LentiKats, ou les mélanges de ceux-ci, en particulier dans l'alginate.

6. Procédé selon au moins l'une des revendications précédentes, **caractérisé en ce qu'**on a dans l'étape B) du procédé un pH de 4 à 9,5.

7. Procédé selon au moins l'une des revendications précédentes, **caractérisé en ce qu'**on a dans l'étape B) du procédé une température de 20 à 40°C.

8. Procédé selon au moins l'une des revendications précédentes, **caractérisé en ce que** le complexe enzymatique immobilisé est utilisé sous forme d'un lit fixe, à travers lequel s'écoule la solution contenant du saccharose.
